# EUROPEAN PATENT SPECIFICATION

(11) **EP 1 581 759 B1**
(45) Date of publication and mention of the grant of the patent: **13.05.2009**
(21) Application number: 03814397.0
(22) Date of filing: 23.12.2003
(51) Int. Cl.: A61K 39/395, C07K 16/28, A61P 25/00

(54) **METHODS FOR TREATING TAXOL-INDUCED SENSORY NEUROPATHY**
VERFAHREN ZUR BEHANDLUNG VON DURCH TAXOL INDUZIERTER SENSORISCHER NEUROPATHIE
METHODES DE TRAITEMENT DE LA NEUROPATHIE SENSORIELLE INDUITE PAR LE TAXOL

(30) Priority: 23.12.2002 US 436147 P
(43) Date of publication of application: 05.10.2005
(73) Proprietor: Rinat Neuroscience Corp., South San Francisco, CA 94080 (US)
(72) Inventor: SHELTON, David, L., Oakland, CA 94618 (US)
(74) Representative: Roques, Sarah Elizabeth
(86) International application number: PCT/US2003/041367
(87) International publication number: WO 2004/058190

(56) References cited:
- WO-A-01/98361
- WO-A2-01/98361
- US-A1- 2002 065 259
- K. POULSEN ET AL.: "Agonist monoclonal antibodies to trkC receptor for treating large fiber sensory neuropathy: Improved drug candidates over NT-3." SOCIETY FOR NEUROSCIENCE ABSTRACTS, vol. 27, no. 1, 2001, page 356, XP008057142 U.S.A.
- W. CAMPANA ET AL.: "Prosaptide prevents paclitaxel neurotoxicity." NEUROTOXICOLOGY, vol. 19, no. 2, April 1998 (1998-04), pages 237-244, XP009008625
- J. HONGO ET AL.: "Agonist monoclonal antibodies to human trkC receptor map to epitopes overlapping with NT-3 binding site." SOCIETY FOR NEUROSCIENCE ABSTRACTS, vol. 27, no. 1, 2001, page 356, XP001053704 U.S.A.
- R. URFER ET AL.: "High resolution mapping of the binding site of TrkA for nerve growth factor and TrkC for neurotrophin-3 on the second immunoglobulin-like domain of the Trk receptors." THE JOURNAL OF BIOLOGICAL CHEMISTRY, vol. 273, no. 10, 6 March 1998 (1998-03-06), pages 5829-5840, XP002116515 Baltimore, MD, USA
- D. SHELTON ET AL.: "Human trks: molecular cloning, tissue distribution, and expression of extracellular domain immunoadhesins." THE JOURNAL OF NEUROSCIENCE, vol. 15, no. 1, January 1995 (1995-01), pages 477-491, XP002358045 New York, NY, USA
- CAMPANA W.M. ET AL.: 'Prosapeptide prevents paclitaxel neurotoxicity' vol. 19, no. 2, April 1998, pages 237 - 244, XP009008625
- POLOMANO R.C. ET AL.: 'Chemotherapy-evoked painful peripheral neuropathy' PAIN MEDICINE vol. 2, no. 1, December 2001, pages 8 - 14, XP002983514

## Description

### FIELD OF THE INVENTION

This invention relates to the field of taxol-induced neuropathy. More specifically, the invention relates to methods of treating taxol-induced sensory neuropathy comprising administration of agonist anti-trkC antibody for the treatment, prevention, and/or amelioration of a symptom of taxol-induced sensory neuropathy.

### BACKGROUND OF THE INVENTION

Chemotherapeutic agents, such as taxol and other taxanes, have been successfully used in treating cancer. Approximately 300,000 people in the U.S. alone will undergo chemotherapy treatment each year for cancers of the breast, lung and colon. However, 60-90% of patients treated with taxol experience symptoms of neuropathy, including sensory neuropathy and neuronal dysfunction. Frequently observed symptoms present in patients treated with taxol include distal symmetrical paraesthesia, pall-hypaesthesia, loss of joint position sense, painful dysaesthesia, Lhermitte's sign, and pain. Other less frequent symptoms are progressive distal and/or proximal paresis, motor neuropathy, myalgia, rare cases of myopathy, paralytic ileus, orthostatic hypotension, and arrhythmia. (Quasthoff et al., J. Neurol. 249: 9-17 (2002)). Severe sensory neuropathic symptoms necessitate reduction in chemotherapy dosing and delays in treatment thereby limiting the effectiveness of anti-cancer therapy.

Neurotrophins are a family of small, homodimeric proteins, which play a crucial role in the development and maintenance of the nervous system. Members of the neurotrophin family include nerve growth factor (NGF), brain-derived neurotrophic factor (BDNF), neurotrophin-3 (NT-3), neurotrophin-4/5 (NT-4/5), neurotrophin-6 (NT-6), and neurotrophin-7 (NT-7). Neurotrophins, similar to other polypeptide growth factors, affect their target cells through interaction with cell surface receptors. According to current knowledge, two kinds of transmembrane glycoproteins serve as receptors for neurotrophins. Neurotrophin-responsive neurons possess a common low molecular weight (65-80 kDa), low affinity receptor (LNGFR), also termed as p75NTR or p75, which binds NGF, BDNF, NT-3 and NT-4/5 with a KD of 2x10⁻⁹ M; and large molecular weight (130-150 kDa), high-affinity (KD in the 10⁻¹¹ M range) receptors, which are members of the trk family of receptor tyrosine kinases. The identified members of the trk receptor family are trkA, trkB, and trkC.

TrkC is widely expressed in the central nervous system, and on a subset of neurons in the peripheral nervous system. It is expressed on sympathetic neurons and on a subset of primary sensory neurons of the dorsal root ganglia (DRG), the large fiber sensory neurons of the DRG. Large fiber sensory neurons have large myelinated axons extending to the periphery, where they convey information regarding proprioception, and fine touch and vibration sense.

The extracellular domains of full-length native trkA, trkB and trkC receptors have five structural domains that have been defined with reference to homologous or otherwise similar structures identified in various other proteins. The domains have been designated starting at the N-terminus of the amino acid sequence of the mature trk receptors as 1) a first cysteine-rich domain; 2) a leucine-rich domain; 3) a second cysteine-rich domain; 4) a first immunoglobulin-like domain; and 5) a second immunoglobulin-like domain. *See, e.g.,* WO 0198361; Urfer et al. J. Biol. Chem. 273: 5829-5840 (1998).

Neurotrophins are of interest as potential therapeutic agents for a variety of neurodegenerative and neurological diseases. Neurotrophins, such as NGF and NT-3, were tested in animal models for treating sensory neuropathy associated with pyridoxine or cis-platinum treatment. U.S. Patent. No. 5,604,202; WO 0198361. Campara et al, Neurotoxicology, 19(2): 237-244 (1998) describe the use of peptides for the neurotrophic sequence of Sapasin C for the treatment of paclitaxel induced neurotoxicity induced neurotoxicity. Using neurotrophins in treatment of neurodegenerative and neurological diseases have several shortcomings. One significant shortcoming is the lack of specificity. Most neurotrophins cross-react with more than one receptor. For example NT-3, the preferred ligand of the trkC receptor tyrosine kinase, also binds to and activates trkA and trkB (Barbacid, J. Neurobiol. 25:1386-1403 (1994); Barbarcid, Ann. New York Aced Sci. 766:442-458 (1995); Ryden and Ibanez, J. Biol. Chem. 271:5623-5627 (1996), Belliveau et al., J. Cell. Biol. 136:375-388 (1997); Farinas et al., Neuron 21:325-334 (1998)). As a result, it is difficult to devise therapies that target a specific population of neurons. Another limitation of neurotrophin therapy is that neurotrophins, including NT-3, are known to elicit hyperalgesia (Ghaudhry et al., Muscle and Nerve 23:189-192 (2000)). In addition, some neurotrophins such as NT-3 have poor pharmacokinetic and bioavailability properties in rodents, which raise serious questions about their human clinical applications (Haase et al., J. Neurol. Sci. 160:S97-S105 (1998), dosages used in Helgren et al., J. Neurosci. 17(1):372-82 (1997)).

Treatment of cancer with chemotherapeutic agents can be associated with nervous system damage and dysfunction. Use of agonist anti-trkC monoclonal antibodies in treatment in animal models for cisplatinum- and pyridoxine-induced sensory neuropathy has been described. U.S. Patent No. 5,910,574; PCT Publication No. WO 019836 and Poulser et al., Soc. Neuroscience Abstracts, 21,1,2001, 356. However, the pathology or symptoms of chemotherapy-induced neuropathies varies with respect to the chemotherapeutic agent used in treating cancer. For example, cisplatinum and pyridoxine have different symptoms and likely different mechanism of nerve damage than does taxol, although all three agents induce neuropathies. For example, cis-platinum is a DNA adduct agent, causing single- and double-stranded crosslinks in DNA. Quasthoff (2002) J. Neurology 249:9-17. By contrast, taxol functions to prevent microtubule depolymerization, resulting in aggregation of intracellular microtubules. For example, neuropathy in individuals treated with taxol is associated with weakness, and other acute symptoms, and taxol treatment affects all sensory modalities, including thick myelinated fibers that conduct vibration, and confer perception and sense of position.
Electrophysiological characterization in humans and animal models having taxol-induced neuropathy reveals an acute effect on sensory function (*e.g*. decreased compound action potential amplitude) and decreased sensory nerve conduction velocity. *See* Quasthoff, *supra;* Cliffer et al., Ann. Neurol. (1998) 43:46-55. By contrast, weakness is infrequently observed in cis-platinum- and pyridoxine-induced neuropathies. Cis-platinum neuropathy results in decreased sensory nerve conduction velocity, but little change in compound sensory action potential amplitude. Pyridoxine treatment results in decreased compound sensory action potential amplitude. *See* Quasthoff, *supra;* PCT Publication No. WO 01/98361. Thus, the neuropathies caused by pyridoxine and cis-platinum display different symptoms from that caused by taxol.

There is a great need for new therapeutic treatment for taxol-induced sensory neuropathy.

### DISCLOSURE OF THE INVENTION

The present invention is based on the discovery that agonist anti-trkC antibodies treat sensory neuropathy present in individuals treated with taxol. Taxol-induced sensory neuropathy refers to a neurological disorder associated with or present in an individual following administration of the agent, taxol, or related taxanes. A taxol-induced sensory neuropathy affects the peripheral nerves, most often manifested as one or a combination of sensory, sensorimotor, or autonomic dysfunction, including degeneration, or other dysfunction of large fiber peripheral sensory neurons. Thus, the present invention encompasses methods of treating, preventing, delaying the development of a symptom of, increasing the rate of recovery from, and/or palliating taxol-induced sensory neuropathy using agonist anti-trkC antibodies.

Accordingly, the invention provides an anti-trkC agonist antibody for treating a taxane induced sensory neuropathy in an individual an anti-trkC agonist antibody may be used to delay development of a symptom associated with a taxol-induced sensory neuropathy in an individual an anti-trkC agonist antibody may be used to ameliorate a symptom of a taxol-induced sensory neuropathy in an individual may be used to reverse a preexisting taxol-induced sensory neuropathy and/or increase the rate of recovery from a preexisting taxol induced sensory neuropathy an anti-trkC agonist antibody may be used to enhance maintenance and/or regeneration of peripheral neurons in an individual having taxol-induced sensory neuropathy.

an anti-trkC agonist antibody may be used to for treating an individual having cancer, in conjunction with taxol. The cancer may be any one or more of: breast cancer, ovarian cancer, lung cancer, Kaposi's sarcoma, prostate cancer, head and neck cancers, and hematological malignancies.

Agonist anti-trkC antibodies are known in the art. In some embodiments, the agonist anti-trkC antibody binds human trkC. In some embodiments, the agonist anti-trkC antibody specifically binds human trkC. The agonist anti-trkC antibody may also bind human and rodent trkC. The agonist anti-trkC antibody may be a human antibody (such as antibody 6.4.1 (PCT Publication No. WO 01/98361)) or may be a humanized antibody (including humanized monoclonal antibody 2256). In another embodiment, the agonist anti-trkC antibody is humanized antibody A5, as described herein. In still other embodiments, the anti-trkC agonist antibody comprises the amino acid sequence of the heavy chain variable region shown in Table 1 (SEQ ID NO:1) and the amino acid sequence of the light chain variable region shown in Table 2 (SEQ ID NO:2). In other embodiments, the anti-trkC agonist antibody comprises one or more CDR(s) of antibody A5 (such as one, two three, four, five or, in some embodiments, all six CDRs from A5). Identification of CDRs is well within the skill of the art. In some embodiments, the CDRs comprise the Kabat CDR. In other embodiments, the CDRs are the Chothia CDR. In still other embodiments, the CDR comprises both the Kabat and Chothia CDRs. In some embodiments, the antibody comprises a light chain that is encoded by a polynucleotide that is produced by a host cell with a deposit number of ATCC No. PTA-5682. In some embodiments, the antibody comprises a heavy chain that is encoded by a polynucleotide that is produced by a host cell with a deposit number of ATCC No. PTA-5683. In some embodiments, the antibody comprises (a) a light chain that is encoded by a polynucleotide that is produced by a host cell with a deposit number of ATCC No. PTA-5682; and (b) a heavy chain that is encoded by a polynucleotide that is produced by a host cell with a deposit number of ATCC No. PTA-5683. In some embodiments, the antibody comprises one or more CDR(s) encoded by (a) a polynucleotide that is produced by a host cell with a deposit number of ATCC No. PTA-5682; and/or (b) a heavy chain that is encoded by a polynucleotide that is produced by a host cell with a deposit number of ATCC No. PTA-5683.

The antibody may bind essentially the same trkC epitope as an antibody selected from any one or more of the following: 6.1.2, 6.4.1, 2345, 2349, 2.5.1, 2344, 2248, 2250, 2253, and 2256. See PCT Publication No. WO 01/98361. The antibody may comprise a modified constant region, such as a constant region that is immunologically inert, *e.g.,* does not trigger a complement mediated lysis or does not stimulate antibody-dependent cell mediated cytotoxicity (ADCC). In other embodiments, the constant region is modified as described in Eur. J. Immunol. (1999) 29:2613-2624; PCT Application No. PCT/GB99/01441; and/or UK Patent Application No. 9809951.8.

The antibody may also be an antibody fragment, such as an antibody fragment selected from one or more of the following: Fab, Fab', F(ab')₂, Fv fragments, diabodies, single chain antibody molecules and multispecific antibodies formed from antibody fragments, and a single-chain Fv (scFv) molecule. The antibody may also be chimeric, and it may be bispecific.

The agonist anti-trkC antibody can be administered prior to, during or after administration of taxol, and/or can be delivered before initiating course of taxol therapy; during a course of taxol therapy, and/or after cessation of a course oftaxol therapy. Administration can be before onset of neuropathy.

Administration of an agonist anti-trkC antibody can be by any suitable method known in the art, including one or more of the following means: intravenously, subcutaneously, via inhalation, intrarterially, intramuscularly, intracardially, intraventricularly, intrathecally, intraspinally, and intraperitoneally. Administration may be systemic (*e.g.* intravenously) and/or localized. Administration may be acute and/or chronic.

In another aspect, the invention provides compositions comprising an agonist anti-trkC antibody for use in treating taxane-induced sensory Neuropathy

Any of the compositions and kits described may be for any use described herein whether in the context of use as medicament and/or use for manufacture of a medicament.

### BRIEF DESCRIPTION OF THE FIGURES

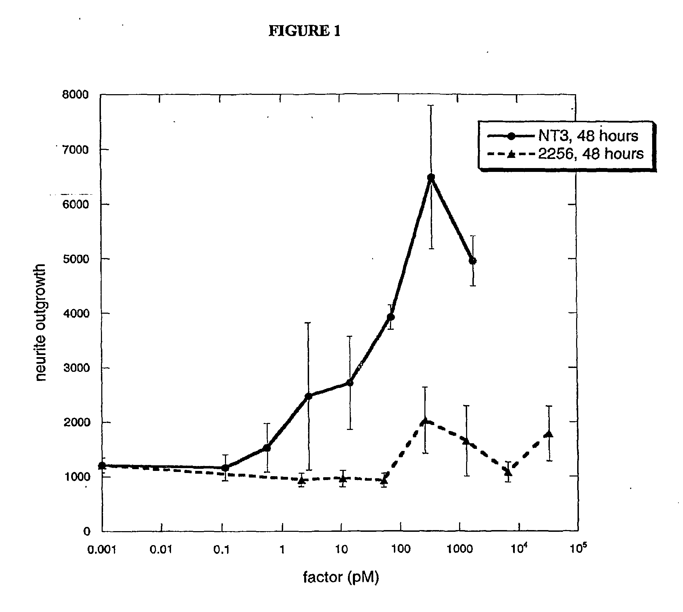

FIGURE 1: is a graph showing that NT3, but not agonist anti-trkC antibodies, caused axon outgrowth of adult sensory neurons. Cultures of rat adult dorsal root ganglion (DRG) neurons were grown in the presence or absence of various concentrations of NT-3 or anti-trkC agonist mouse monoclonal antibody 2256. *See* PCT Publication No. WO O1/98361. After culture for 48 hours, cultures were fixed and stained with RT97 antibodies, and neurite outgrowth was assessed in RT97+ neurons. Treatment with NT-3 caused a dramatic increase in neurite outgrowth, while treatment with anti-trkC agonist antibody did not result in significant outgrowth.

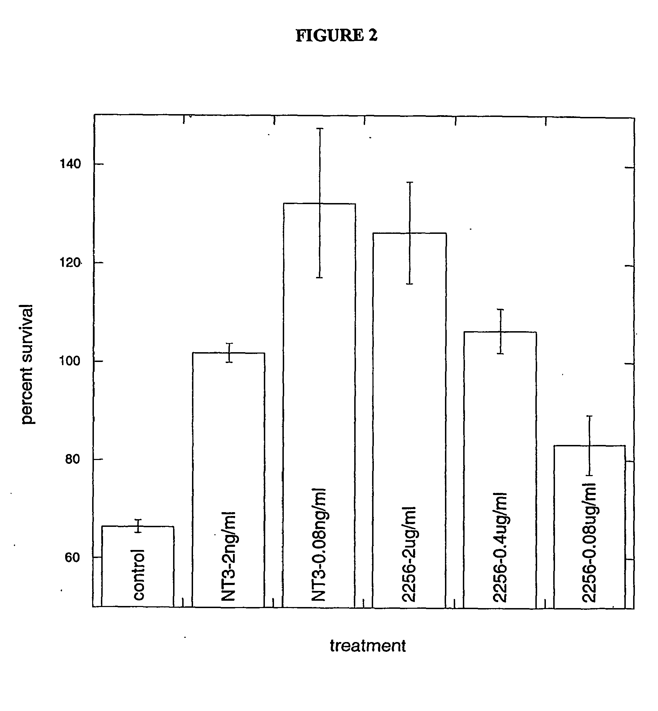

FIGURE 2: is a graph showing survival of rat E12 trigeminal sensory neurons in the presence of NT3 or agonist anti-trkC antibody 2256. To quantify the number of neurons surviving under different experimental conditions the total number of neurons was counted 4-6 hours after plating and again after 24 and 48 hours. The number of neurons at 24 and 48 hours is expressed as a percentage of the 6-hour count.

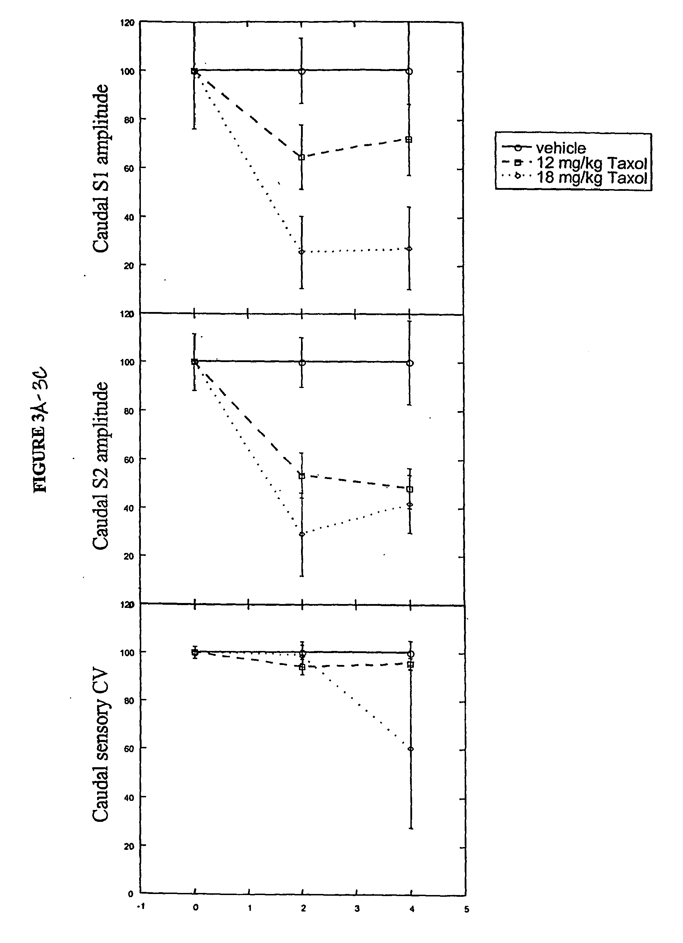

FIGURES 3A-3C: are graphs showing development and dose dependence of taxol-induced sensory neuropathy, as measured by electrophysiological recording in the rat caudal nerve. Rats were treated by slow IV infusion with taxol at 12 or 18 mg/kg or control (vehicle) on days 1 and 4. Electrophysiological recording was performed on days 0, 14, and 28 as described herein.

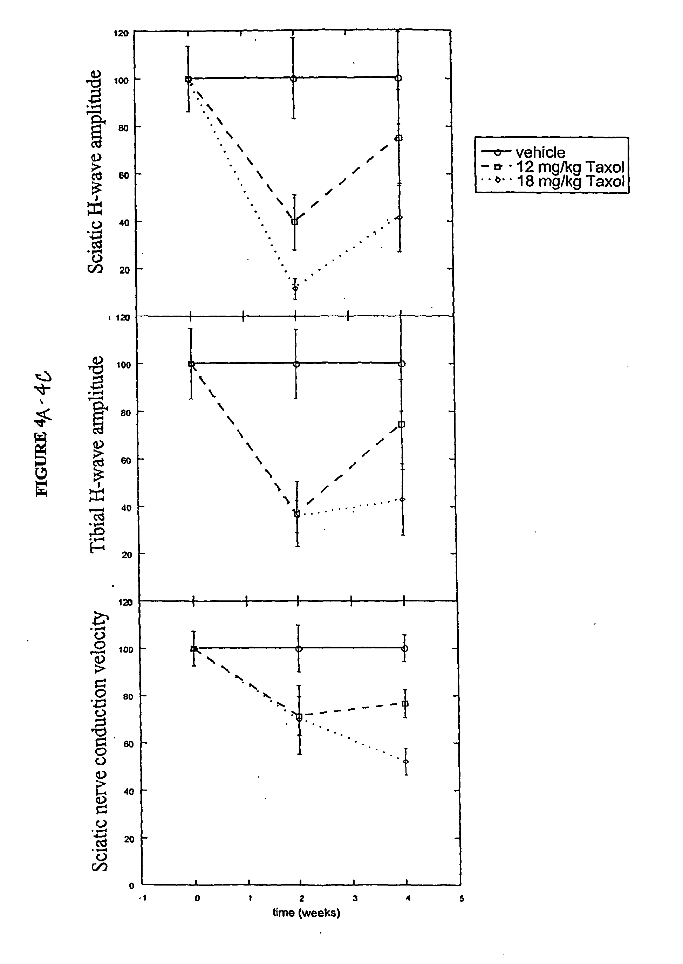

FIGURES 4A-4C: are graphs showing development and dose dependence of taxol-indueed sensory neuropathy, as measured by electrophysiological recording in the rat sciatic nerve. Rats were treated by slow IV infusion with taxol at 12 or 18 mg/kg or vehicle on days 1 and 4. Electrophysiological recording was performed on days 0, 14, and 28 as described herein.

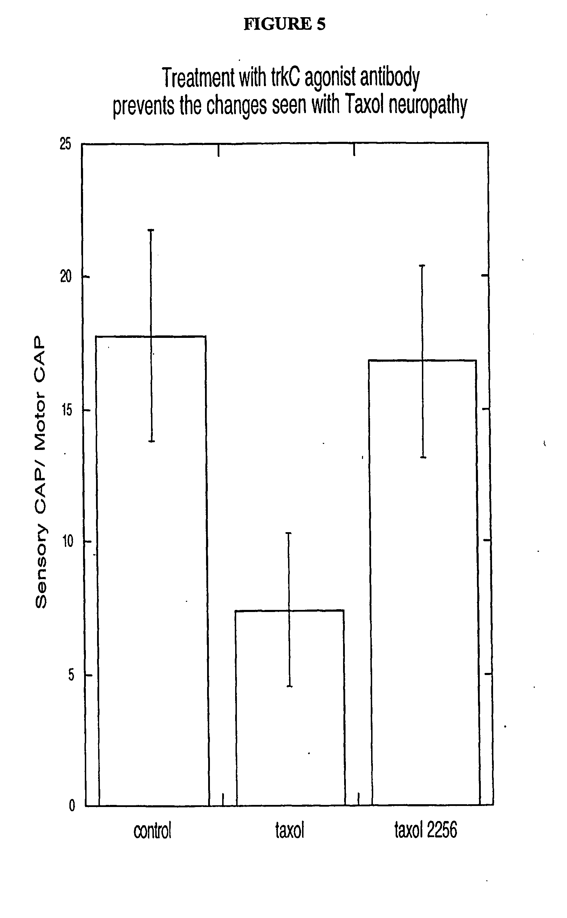

FIGURE 5: is a graph showing that treatment with an anti-trkC agonist antibody ameliorated sensory neuropathy induced by taxol treatment in a rat model. Rats were treated with intravenous (IV) mouse monoclonal antibody 2256 at 5 mg/kg on days 0 and day 7. Taxol was given by slow IV infusion twice, on days 1 and 4, at a dose of 1 S mg/kg. Electrophysiological recording was performed on the caudal nerve on day 14. Data are expressed as the ratio of the sensory compound action potential amplitude to the motor compound action potential amplitude.

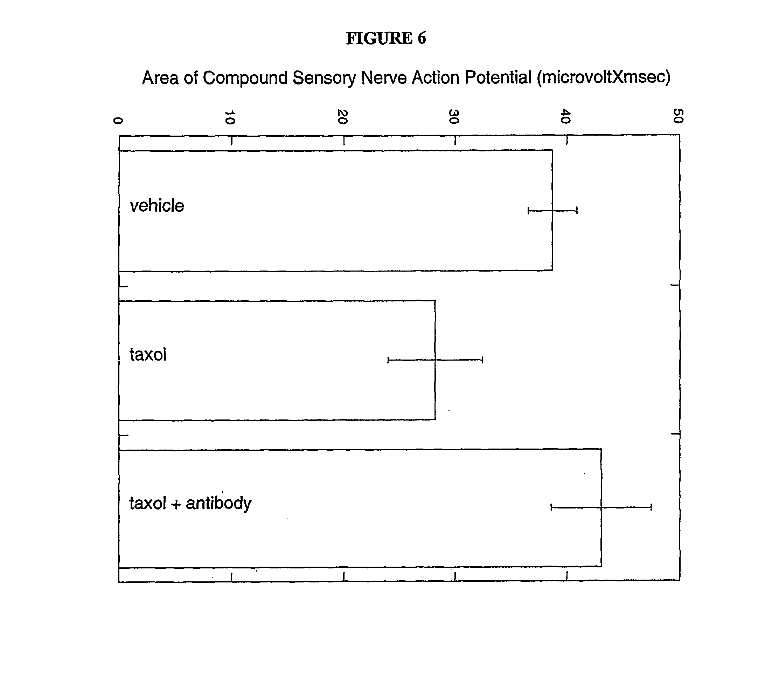

FIGURE 6: is a graph showing that treatment with an anti-trkC agonist antibody ameliorated sensory neuropathy induced by taxol treatment in a mouse model. Mice were treated with mouse monoclonal antibody 2256 subcutaneously (delivered under the scruff) at 2 mg/kg on days 0 and day 7. Taxol was given intraperitoneally (IP) on days 1, 3, and 5, at a total dose of 300 mg/m², split evenly into the three doses. Caudal nerve sensory function was assessed by electrophysiological recording performed on day 14 as described herein. The results are shown as the ratio of the sensory compound action potential amplitude to the motor compound action potential amplitude.

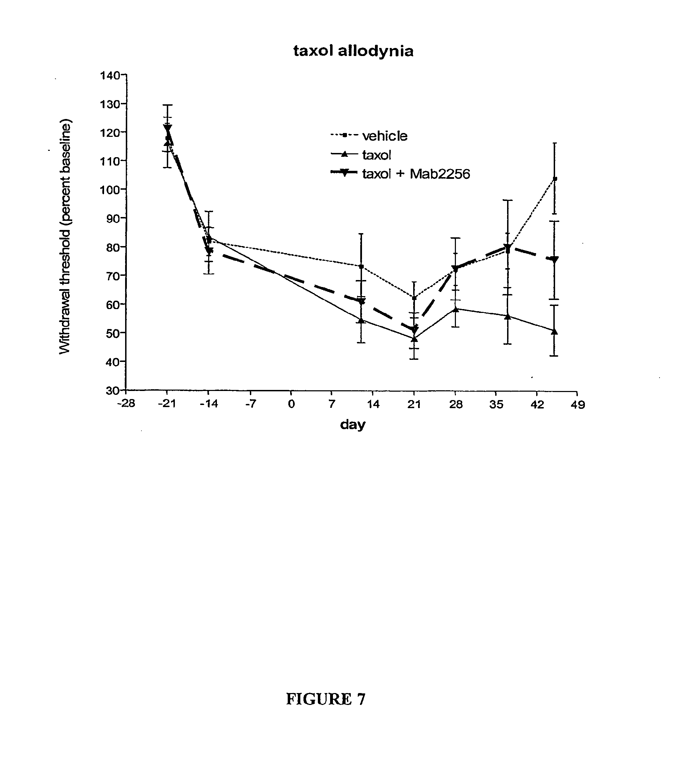

FIGURE 7: is a graph showing neuropathic pain (mechanical allodynia) in response to mechanical stimulation as assessed at various times before and after taxol treatment. Mechanical allodynia developed in animals treated with taxol. Rats treated with taxol alone developed a significant and long-lasting decrease in the response threshold of animals, indicating allodynia (p < 0.0001 using 2-ay ANOVA analysis). Cotreatment of animals with taxol and agonist anti-trkC antibody decreased the depth of allodynia observed with taxol treated animals, and dramatically increased the speed of recovery from the allodynia (p< 0.05, using 2-way ANOVA analysis).

### MODES FOR CARRYING OUT THE INVENTION

The present invention is based on the discovery that agonist anti-trkC antibodies treat sensory neuropathy present in individuals treated with taxol. Taxol-induced sensory neuropathy refers to a neurological disorder affecting the sensory neurons associated with or present in an individual (including a mammal, both human and non-human) following administration of the agent, taxol, or related taxanes. The present invention encompasses methods and compositions useful for treating, preventing, delaying the development of a symptom of, increasing the rate of recovery from, and/or palliating taxol-induced sensory neuropathy using agonist anti-trkC antibodies.

Accordingly, in one aspect, the invention relates to treating a taxol-induced sensory neuropathy in an individual comprising administering an effective amount of agonist anti-trkC antibody. Methods of delaying development of a symptom associated with a taxol-induced sensory neuropathy in an individual may comprise treatment of that individual with an effective amount of agonist anti-trkC antibody. Methods of ameliorating a symptom of a taxol-induced sensory neuropathy in an individual may comprise administering an effective amount of agonist anti-trkC antibody. Methods for reversing and/or increasing the rate of recovery from a preexisting taxol induced sensory neuropathy may be by treatment with an effective amount of agonist anti-trkC antibody. Maintenance and/or regeneration of peripheral neurons in an individual having taxol-induced sensory neuropathy may be enhanced. The treatment of cancer with taxol may be enhanced as described herein, by administration of taxol in conjunction with an agonist anti-trkC antibody.

The agonist anti-trkC antibody can be administered prior to, during and/or after administration of taxol. Alternately, the antibody can be administered in conjunction with the taxol. The agonist anti-trkC antibody can be can be administered prior to, during and/or after administration of any other therapeutic modality for the sensory neuropathy. The antibody can be administered in conjunction with any other therapeutic modality for the sensory neuropathy.

Administration of an agonist anti-trkC antibody can be by any means known in the art, including one or more of the following means: intravenously, subcutaneously, via inhalation, intrarterially, intramuscularly, intracardially, intraventricularly, intrathecally, and intraperitoneally. Administration may be systemic (*e.g*. intravenously), or localized. Administration may be acute or chronic.

In another aspect, compositions and kits comprising an agonist anti-trkC antibody may be used in any of the methods described herein

Any of the compositions described may be used described herein whether in the context of use a s medicament and/or use for manufacture of a medicament.

### General Techniques

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al.,1989) Cold Spring Harbor Press; Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds.,1993-8) J. Wiley and Sons; Methods in Enzymology (Academic Press, Inc.); Handbook of Experimental Immunology (D.M. Weir and C.C. Blackwell, eds.); Gene Tranfer Vectors for Mammalian Cells (J.M. Miller and M.P. Calos, eds., 1987); Current Protocols in Molecular Biology (F.M. Ausubel et al., eds., 1987); PCR: The Polymerase Chain Reaction (Mullis et al., eds., 1994); Current Protocols in Immunology (J.E. Coligan et al., eds.,1991); Short Protocols in Molecular Biology (Wiley and Sons,1999); Immunobiology (C.A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J.D. Capra, eds., Harwood Academic Publishers, 1995); and Cancer: Principles and Practice of Oncology (V.T. DeVita et al., eds., J.B. Lippincott Company, 1993).

### Definitions

An "antibody" (interchangeably used in plural form) is an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term encompasses not only intact polyclonal or monoclonal antibodies, but also fragments thereof (such as Fab, Fab', F(ab')₂, Fv), single chain (ScFv), mutants thereof, fusion proteins comprising an antibody portion, humanized antibodies, chimeric antibodies, diabodies linear antibodies, single chain antibodies, multispecific antibodies (*e.g.,* bispecific antibodies) and any other modified configuration of the immunoglobulin molecule that comprises an antigen recognition site of the required specificity. An antibody includes an antibody of any class, such as IgG, IgA, or IgM, and the antibody need not be of any particular class. Depending on the antibody amino acid sequence of the constant domain of its heavy chains, immunoglobulins can be assigned to different classes. There are five major classes of immunoglobulins: IgA, IgG, IgD, IgE, and IgM, and several of these may be further divided into subclasses (isotypes), *e.g.,* IgG1, IgG2, IgG3, IgG4, IgA1 and IgA2. The heavy-chain constant domains that correspond to the different classes of immunoglobulins are called alpha, gamma, delta, epsilon, and mu, respectively. There are also two classes of light chain, designated kappa and lambda. The subunit structures and three-dimensional configurations of different classes of immunoglobulins are well known.

A "monoclonal antibody" refers to a homogeneous antibody population wherein the monoclonal antibody is comprised of amino acids (naturally occurring and non-naturally occurring) that are involved in the selective binding of an antigen. A population of monoclonal antibodies is highly specific, being directed against a single antigenic site. The term "monoclonal antibody" encompasses not only intact monoclonal antibodies and full-length monoclonal antibodies, but also fragments thereof (such as Fab, Fab', F(ab')₂, Fv), single chain (ScFv), mutants thereof, fusion proteins comprising an antibody portion, humanized monoclonal antibodies, chimeric monoclonal antibodies, and any other modified configuration of the immunoglobulin molecule that comprises an antigen recognition site of the required specificity and the ability to bind to an antigen. It is not intended to be limited as regards to the source of the antibody or the manner in which it is made (*e.g.,* by hybridoma, phage selection, recombinant expression, transgenic animals, etc.).

"Humanized" antibodies refer to a molecule having an antigen binding site that is substantially derived from an immunoglobulin from a non-human species and the remaining immunoglobulin structure of the molecule based upon the structure and/or sequence of a human immunoglobulin. The antigen binding site may comprise either complete variable domains fused onto constant domains or only the complementarity determining regions (CDRs) grafted onto appropriate framework regions in the variable domains. Antigen binding sites may be wild type or modified by one or more amino acid substitutions, *e.g.,* modified to resemble human immunoglobulin more closely. Some forms of humanized antibodies preserve all CDR sequences (for example, a humanized mouse antibody which contains all six CDRs from the mouse antibodies). Other forms of humanized antibodies have one or more CDRs (one, two, three, four, five, six) which are altered with respect to the original antibody.

As used herein, "human antibody" means an antibody having an amino acid sequence corresponding to that of an antibody produced by a human and/or has been made using any of the techniques for making human antibodies known in the art or disclosed herein. This definition of a human antibody includes antibodies comprising at least one human heavy chain polypeptide or at least one human light chain polypeptide. One such example is an antibody comprising murine light chain and human heavy chain polypeptides. Human antibodies can be produced using various techniques known in the art. In one embodiment, the human antibody is selected from a phage library, where that phage library expresses human antibodies (Vaughan et al., 1996, Nature Biotechnology, 14:309-314; Sheets et al., 1998, PNAS, (USA) 95:6157-6162; Hoogenboom and Winter, 1991, J. Mol. Biol., 227:381; Marks et al., 1991, J. Mol. Biol., 222:581). Human antibodies can also be made by introducing human immunoglobulin loci into transgenic animals, *e.g.,* mice in which the endogenous immunoglobulin genes have been partially or completely inactivated. This approach is described in U.S. Patent Nos. 5,545,807; 5,545,806; 5,569,825; 5,625,126; 5,633,425; and 5,661,016. Alternatively, the human antibody may be prepared by immortalizing human B lymphocytes that produce an antibody directed against a target antigen (such B lymphocytes may be recovered from an individual or may have been immunized *in vitro). See, e.g.,* Cole et al., Monoclonal Antibodies and Cancer Therapy, Alan R. Liss, p. 77 (1985); Boerner et al., 1991, J. Immunol., 147 (1):86-95; and U.S. Patent No. 5,750,373.

"Chimeric antibodies" refers to those antibodies wherein one portion of each of the amino acid sequences of heavy and light chains is homologous to corresponding sequences in antibodies derived from a particular species or belonging to a particular class, while the remaining segment of the chains is homologous to corresponding sequences in another. Typically, in these chimeric antibodies, the variable region of both light and heavy chains mimics the variable regions of antibodies derived from one species of mammals, while the constant portions are homologous to the sequences in antibodies derived from another. One clear advantage to such chimeric forms is that, for example, the variable regions can conveniently be derived from presently known sources using readily available hybridomas or B cells from non human host organisms in combination with constant regions derived from, for example, human cell preparations. While the variable region has the advantage of ease of preparation, and the specificity is not affected by its source, the constant region being human, is less likely to elicit an immune response from a human subject when the antibodies are injected than would the constant region from a non-human source. However, the definition is not limited to this particular example.

An epitope that "specifically binds" or "preferentially binds" (used interchangeably herein) to an antibody or a polypeptide is a term well understood in the art, and methods to determine such specific or preferential binding are also well known in the art. A molecule is said to exhibit "specific binding" or "preferential binding" if it reacts or associates more frequently, more rapidly, with greater duration and/or with greater affinity with a particular cell or substance than it does with alternative cells or substances. An antibody "specifically binds" or "preferentially binds" to a target if it binds with greater affinity, avidity, more readily, and/or with greater duration than it binds to other substances. For example, an antibody that specifically or preferentially binds to a trkC epitope is an antibody that binds this trkC epitope with greater affinity, avidity, more readily, and/or with greater duration than it binds to other trkC epitopes or non-trkC epitopes. It is also understood by reading this definition that, for example, an antibody (or moiety or epitope) that specifically or preferentially binds to a first target may or may not specifically or preferentially bind to a second target. As such, "specific binding" or "preferential binding" does not necessarily require (although it can include) exclusive binding. Generally, but not necessarily, reference to binding means preferential binding.

A "functional Fc region" possesses at least one effector function of a native sequence Fc region. Exemplary "effector functions" include C1q binding; complement dependent cytotoxicity (CDC); Fc receptor binding; antibody-dependent cell-mediated cytotoxicity (ADCC); phagocytosis; down-regulation of cell surface receptors (*e.g*. B cell receptor; BCR), etc. Such effector functions generally require the Fc region to be combined with a binding domain (*e.g*. an antibody variable domain) and can be assessed using various assays known in the art for evaluating such antibody effector functions.

A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification, yet retains at least one effector function of the native sequence Fc region. Preferably, the variant Fc region has at least one amino acid substitution compared to a native sequence Fc region or to the Fc region of a parent polypeptide, *e.g*. from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95% sequence identity therewith.

As used herein "antibody-dependent cell-mediated cytotoxicity" and "ADCC" refer to a cell-mediated reaction in which nonspecific cytotoxic cells that express Fc receptors (FcRs) (*e.g*. natural killer (NK) cells, neutrophils, and macrophages) recognize bound antibody on a target cell and subsequently cause lysis of the target cell. ADCC activity of a molecule of interest can be assessed using an *in vitro* ADCC assay, such as that described in U.S. Patent No. 5,500,362 or 5,821,337. Useful effector cells for such assays include peripheral blood mononuclear cells (PBMC) and NK cells. Alternatively, or additionally, ADCC activity of the molecule of interest may be assessed *in vivo, e.g.,* in an animal model such as that disclosed in Clynes et al., 1998, PNAS (USA), 95:652-656.

An "agonist anti-trkC antibody" (interchangeably termed "anti-trkC agonist antibody") refers to an antibody that is able to bind to and activate a trkC receptor and/or downstream pathway(s) mediated by the trkC signaling function. For example, the agonist antibody may bind to the extracellular domain of a trkC receptor and thereby cause dimerization of the receptor, resulting in activation of the intracellular catalytic kinase domain. Consequently, this may result in stimulation of growth and/or differentiation of cells expressing the receptor in vitro and/or in vivo. In some embodiments, an agonist anti-trkC antibody binds to trkC and activates a trkC biological activity. In some embodiments, an agonist antibody useful in the methods of the invention recognizes domain V and/or domain IV oftrkC. *See* Urfer et al., J. Biol. Chem. 273: 5829-5840 (1998).

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen binding site of antibodies. There are at least two techniques for determining CDRs: (1) an approach based on cross-species sequence variability (*i.e.,* Kabat et al. Sequences of Proteins of Immunological Interest, (5th ed., 1991, National Institutes of Health, Bethesda MD)); and (2) an approach based on crystallographic studies of antigen-antibody complexes (Al-lazikani et al (1997) J. Molec. Biol. 273:927-948)). As used herein, a CDR may refer to CDRs defined by either approach or by a combination of both approaches.

A "constant region" of an antibody refers to the constant region of the antibody light chain or the constant region of the antibody heavy chain, either alone or in combination.

As used herein, "Fc receptor" and "FcR" describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. FcRs are reviewed in Ravetch and Kinet, 1991, Ann. Rev. Immunol., 9:457-92; Capel et al., 1994, Immunomethods, 4:25-34; and de Haas et al., 1995, J. Lab. Clin. Med., 126:330-41. "FcR" also includes the neonatal receptor, FcRn, which is responsible for the transfer of maternal IgGs to the fetus (Guyer et al., 1976, J. Immunol., 117:587; and Kim et al., 1994, J. Immunol., 24:249).

"Complement dependent cytotoxicity" and "CDC" refer to the lysing of a target in the presence of complement. The complement activation pathway is initiated by the binding of the first component of the complement system (C1q) to a molecule (*e.g.* an antibody) complexed with a cognate antigen. To assess complement activation, a CDC assay, *e.g.* as described in Gazzano-Santoro et al., J. Immunol. Methods, 202:163 (1996), may be performed.

As used herein, "affinity matured" antibody means an antibody with one or more alterations in one or more CDRs thereof that result an improvement in the affinity of the antibody for antigen compared to a parent antibody that does not possess the alteration(s). In some embodiments, affinity matured antibodies will have nanomolar or even picomolar affinities for the target antigen. Affinity matured antibodies are produced by procedures known in the art (Marks et al., 1992, Bio/Technology, 10:779-783; Barbas et al., 1994, Proc Nat. Acad Sci, USA 91:3809-3813; Schier et al., 1995, Gene, 169:147-155; Yelton et al., 1995, J. Immunol., 155:1994-2004; Jackson et al., 1995, J. Immunol., 154(7):3310-9; Hawkins et al, 1992, J. Mol. Biol., 226:889-896).

As used herein, "trkC" refers to the trkC receptor polypeptide, a member of the tyrosine kinase superfamily. TrkC encompasses the native trkC receptor of any mammalian species, including but not limited to, human, canine, feline, bovine, equine, primate, and rodent (including mouse and rat). The extracellular domain of full-length native trkC has been defined with reference to homologous or otherwise similar structures identified in various other proteins. The domains have been designated starting at the N-terminus of the mature trkC receptor as: 1) a first cysteine-rich domain extending from amino acid 1 to amino acid 48; 2) a leucine-rich domain extending from amino acid 49 to amino acid 120; 3) a second cysteine-rich domain extending from amino acid 121 to amino acid 177; 4) a first immunoglobulin-like domain extending from about amino acid 196 to amino acid 257; and 5) a second immunoglobulin-like domain extending from about amino acid 288 to amino acid 351. *See, e.g.,* PCT Publication No. WO 0198361. The domain structure of the human trkC receptor has also been designated by reference to a crystal structure as follows: domain 1 from amino acid 1 to amino acid 47; domain 2 from amino acid 48 to amino acid 130; domain 3 from amino acid 131 to amino acid 177; domain 4 from amino acid 178 to amino acid 165; and domain 5 from amino acid 166 to amino acid 381. *See, e.g.,* PCT Publication No. WO 0198361; Urfer et al. J. Biol. Chem. 273: 5829-5840 (1998). Also included are variants oftrkC, examples of which include, but are not limited to, variants without a kinase domain (Shelton, et al., J.Neurosci. 15(1):477-491 (1995)), and variants with a modified kinase domain (Shelton, et al., J.Neurosci. 15(1):477-491 (1995)).

"Biological activity", when used in conjunction with the agonist anti-trkC antibodies of the present invention, generally refers to having the ability to bind and activate the trkC receptor tyrosine kinase and/or a downstream pathway mediated by the trkC signaling function. As used herein, "biological activity" encompasses one or more effector functions in common with those induced by action of NT-3, the native ligand of trkC, on a trkC-expressing cell. A "biological activity" of trkC can also encompass downstream signaling pathway(s) or effector functions that are different than those induced by action of NT-3. Without limitation, biological activities include any one or more of the following: ability to bind and activate trkC; ability to promote trkC receptor dimerization; the ability to promote the development, survival, function, maintenance and/or regeneration of cells (including damaged cells), in particular neurons in vitro or in vivo, including peripheral (sympathetic, sensory, and enteric) neurons, and central (brain and spinal cord) neurons, and non-neuronal cells, *e.g.* peripheral blood leukocytes. A particular preferred biological activity is the ability to treat (including prevention of) one or more symptoms of taxol-induced sensory neuropathy, and/or repair and/or improve the function of a sensory nerve cell damaged by taxol. Exemplary damaged neurons include any of sensory (including large-fiber sensory neurons), sympathetic, or enteric, *e.g.,* dorsal root ganglia neurons, cranial ganglia neurons, and central neurons, *e.g.,* neurons from the spinal cord.

A "taxol-induced sensory neuropathy" is a neurological disorder resulting from treatment with the chemotherapeutic agent taxol or other taxanes. As used herein, "taxol-induced sensory neuropathy" refers to and includes any one or more symptoms associate with this neurological disorder. Taxol-induced sensory neuropathy may affect primary sensory neurons of various types, autonomic neurons comprising sympathetic neurons, and neurons of specialized sensation, such as gustatory, olfactory, acoustic, and vestibular. As used herein, a "taxol-induced sensory neuropathy" refers to a neurological disorder affecting the sensory neurons associated with or present in an individual during or following administration of the agent, taxol, or related taxanes. In some embodiments, "taxol-induced sensory neuropathy" is characterized by degeneration of peripheral sensory neurons (including large-fiber sensory neurons). In some embodiments, "taxol-induced sensory neuropathy" is characterized by any of the following: distal symmetrical paraesthesia, pall-hypaesthesia, loss of joint position sense, painful dysaesthesia, Lhermitte's sign, pain, progressive distal and/or proximal paresis, myalgia, paralytic ileus, orthostatic hypotension, and arrhythmia; and degeneration of peripheral sensory neurons (including large-fiber sensory neurons). These may be determined by a standard neurological examination, patient interview, or more specialized quantitative testing. These more specialized quantitative tests may include, but are not limited to, determination of conduction velocity of the affected neurons by, *e.g*. use of microneurography or other electrophysiological testing; quantitative and/or quantitative determination of ability to sense cutaneous stimulation, including, but not limited to, heat, light touch, vibration, or two point discrimination; tests of hearing; specialized tests of balance; specialized tests of proprioception, or kinesthetic sense; tests of autonomic function, including, but not limited to, test of blood pressure control; and tests of heart rate response to various physiological and pharmacological stimuli. These tests may also include tests of motor skill.

As used herein, "taxol" refers to paclitaxel (TAXOL®, Bristol-Myers Squibb Oncology, Princeton, NJ), docetaxel (TAXOTERE®, Rhône-Poulenc Rorer, Antony, France), and other taxanes. Taxol (including other taxanes) may be administered either alone, or in combination with other drugs. Taxol is approved for and commonly used for treating various malignancies, including Kaposi's sarcoma and those of the breast, ovary, and lung. Taxol is also used to treat other malignancies of the prostate, head and neck, as well as various hematological malignancies. Taxol is also given during bone marrow transplants.

As used herein, "treatment" is an approach for obtaining beneficial or desired clinical results. For purposes of this invention, beneficial or desired clinical results include, but are not limited to, one or more of the following: alleviation of one or more symptoms associated with taxol-induced sensory neuropathy (*e.g.,* any of distal symmetrical paraesthesia, pall-hypaesthesia, loss of joint position sense, loss of vibration sense, loss of two point discrimination, loss of fine touch, uncomfortable dysaesthesia, Lhermitte's sign, pain (including allodynia and/or hyperalgesia), peripheral nerve (including sensory neuron) degeneration); diminishment of extent of a taxol-induced sensory neuropathy; stabilized (i.e., not worsening) state of a taxol-induced sensory neuropathy; preventing occurrence or recurrence of a taxol-induced sensory neuropathy; delaying the development of a taxol-induced sensory neuropathy; delay or slowing of progression of a taxol-induced sensory neuropathy; amelioration of a taxol-induced sensory neuropathy; and remission (whether partial or total) of a taxol-induced sensory neuropathy; increasing the rate of recovery from a taxol-induced sensory neuropathy; reduction of incidence of a taxol-induced sensory neuropathy and/or symptoms associated with a taxol-induced sensory neuropathy.

"Palliating" a taxol-induced sensory neuropathy or one or more symptoms of a taxol-induced sensory neuropathy means lessening the extent and/or time course of undesirable clinical manifestations of a taxol-induced sensory neuropathy in an individual or population of individuals treated with an agonist anti-trkC antibody in accordance with the invention.

"Reducing severity of a symptom" or "ameliorating a symptom" of a taxol-induced sensory neuropathy means a lessening and/or improvement of one or more symptoms of a taxol-induced sensory neuropathy as compared to not administering an agonist anti tpkC antibody. "Reducing severity" also includes shortening or reduction in duration of a symptom. Symptoms of a taxol-induced sensory neuropathy such as distal symmetrical paraesthesia, pall-hypaesthesia, loss of joint position sense, loss of vibration sense, loss of two point discrimination, loss of fine touch, loss of balance, loss or other dysfunction of hearing, uncomfortable dysaesthesia, Lhermitte's sign, and/or pain (including allodynia and/or hyperalgesia) are described *supra.*

As used herein, "delaying" development of a taxol-induced sensory neuropathy means to defer, hinder, slow, retard, stabilize, and/or postpone development of the taxol-induced sensory neuropathy. This delay can be of varying lengths of time, depending on the history of the taxol-induced sensory neuropathy and/or individual being treated. As is evident to one skilled in the art, a sufficient or significant delay can, in effect, encompass prevention, in that the individual does not develop the taxol-induced sensory neuropathy. A method that "delays" development of a taxol-induced sensory neuropathy is a method that reduces probability of development of the sensory neuropathy in a given time frame and/or reduces extent of the sensory neuropathy in a given time frame, when compared to not using the method. Such comparisons are typically based on clinical studies, using a statistically significant number of subjects.

"Development" of a taxol-induced sensory neuropathy means the onset and/or progression of a taxol-induced sensory neuropathy within an individual. A taxol-induced sensory neuropathy development can be detectable using standard clinical techniques as described herein. However, development also refers to disease progression that may be initially undetectable. For purposes of this invention, progression refers to the biological course of the disease state, in this case, as determined by a standard neurological examination, or patient interview or may be determined by more specialized quantitative testing. These more specialized quantitative tests may include, but are not limited to, determination of conduction velocity of the affected neurons by means including microneurography, quantitative determination of ability to sense cutaneous stimulation, including, but not limited to, heat, light touch, vibration, or two point discrimination, tests of hearing, specialized tests of balance, tests of reflexes, specialized tests of proprioception, or kinesthetic sense, tests of autonomic function, including, but not limited to, test of blood pressure control, tests of heart rate response to various physiological and pharmacological stimuli. These tests may also include tests of motor skill. "Development" includes occurrence, recurrence, and onset. As used herein "onset" or "occurrence" of a taxol-induced sensory neuropathy includes initial onset and-and/or recurrence.

As used herein, an "at risk" individual is an individual who is at risk of development of taxol-induced sensory neuropathy. An individual "at risk" may or may not have detectable disease, and may or may not have displayed detectable disease prior to the treatment methods described herein. "At risk" denotes that an individual has one or more so-called risk factors, which are measurable parameters that correlate with development of taxol-induced sensory neuropathy. An individual having one or more of these risk factors has a higher probability of developing taxol-induced sensory neuropathy than an individual without these risk factor(s).

An "effective amount" (in the taxol-induced sensory neuropathy context) is an amount sufficient to effect beneficial or desired clinical results including clinical results or delaying the onset of the disease. An effective amount can be administered in one or more administrations. For purposes of this invention, an effective amount of an agonist anti-trkC antibody described herein is an amount sufficient to ameliorate, stabilize, reverse, slow and/or delay progression of or prevent taxol-induced sensory neuropathy. An effective amount of an agonist anti-trkC antibody also encompasses an amount of an agonist anti-trkC antibody sufficient to enhance taxol treatment (therapeutic effect) of cancer (which can, in turn, mean that taxol dosage is increased and/or some other beneficial effect is observed such as reduction of side-effects of taxol treatment), as described herein. As is understood in the art, an effective amount of an agonist anti-trkC antibody may vary, depending on, inter alia, patient history as well as other factors such as the type (and/or dosage) of an agonist anti-trkC antibody used.

As used herein, administration "in conjunction" includes simultaneous administration and/or administration at different times. Administration in conjunction also encompasses administration as a co-formulation (*e.g.,* an agonist anti-trkC antibody and taxol are present in the same composition) or administration as separate compositions. As used herein, administration in conjunction is meant to encompass any circumstance wherein an agonist anti-trkC antibody and taxol are administered to an individual, which can occur simultaneously and/or separately. As further discussed herein, it is understood that an agonist anti-trkC antibody and taxol can be administered at different dosing frequencies or intervals. For example, an agonist anti-trkC antibody can be administered weekly, while taxol can be administered less frequently. It is understood that the agonist anti-trkC antibody and taxol can be administered using the same route of administration or different routes of administration.

Taxol treatment is "enhanced" when an aspect of taxol treatment is improved (as compared to administering taxol without administering an agonist anti-trkC antibody). For example, the presence and/or intensity of undesired side-effects (such as sensory neuropathy) may be reduced and/or eliminated in the presence of an agonist anti-trkC antibody relative to the presence and/or intensity of such side-effects in the absence of an agonist anti-trkC antibody. This enhancement is indicated by administration of an anti-trkC agonist antibody and is not meant to convey that such a comparison (administration of an anti-trkC agonist antibody versus no administration) must be conducted and proven with respect to any given individual.

An "individual" is a vertebrate, preferably a mammal, more preferably a human. Mammals include, but are not limited to, farm animals, sport animals, pets, primates, horses, cows, cats, dogs, and rodents (such as mice and rats).

As used herein, the singular form "a", "an", and "the" includes plural references unless indicated otherwise. For example, "an" antibody includes one or more antibodies and "a symptom" means one or more symptoms.

As used herein, "vector" means a construct, which is capable of delivering, and preferably expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

As used herein, "expression control sequence" means a nucleic acid sequence that directs transcription of a nucleic acid. An expression control sequence can be a promoter, such as a constitutive or an inducible promoter, or an enhancer. The expression control sequence is operably linked to the nucleic acid sequence to be transcribed.

As used herein, "nucleic acid" or "polynucleotide" refers to a deoxyribonucleotide or ribonucleotide polymer in either single- or double-stranded form, and unless otherwise limited, encompasses known analogs of natural nucleotides that hybridize to nucleic acids in a manner similar to naturally-occurring nucleotides.

As used herein, "pharmaceutically acceptable carrier" includes any material which, when combined with an active ingredient, allows the ingredient to retain biological activity and is non-reactive with the subject's immune system. Examples include, but are not limited to, any of the standard pharmaceutical carriers such as a phosphate buffered saline solution, water, emulsions such as oil/water emulsion, and various types of wetting agents. Preferred diluents for aerosol or parenteral administration are phosphate buffered saline or normal (0.9%) saline.

Compositions comprising such carriers are formulated by well known conventional methods (*see,* for example, Remington's Pharmaceutical Sciences, 18th edition, A. Gennaro, ed., Mack Publishing Co., Easton, PA, 1990; and Remington, The Science and Practice of Pharmacy 20th Ed. Mack Publishing, 2000).

As used herein, "adjuvant" includes those adjuvants commonly used in the art to facilitate an immune response. Examples of adjuvants include, but are not limited to, helper peptide; aluminum salts such as aluminum hydroxide gel (alum) or aluminum phosphate; Freund's Incomplete Adjuvant and Complete Adjuvant (Difco Laboratories, Detroit, MI); Merck Adjuvant 65 (Merck and Company, Inc., Rahway, NJ); AS-2 (Smith-Kline Beecham); QS-21 (Aquilla Biopharmaceuticals); MPL or 3d-MPL (Corixa Corporation, Hamilton, MT); LEIF; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A; muramyl tripeptide phosphatidyl ethanolamine or an immunostimulating complex, including cytokines (*e.g.,* GM-CSF or interleukin-2, -7 or -12) and immunostimulatory DNA sequences. In some embodiments, such as with the use of a polynucleotide vaccine, an adjuvant such as a helper peptide or cytokine can be provided via a polynucleotide encoding the adjuvant.

### Uses of the Invention

With respect to all methods and uses described herein, reference to agonist anti-trkC antibodies also includes compositions comprising one or more of these antibodies. These compositions may further comprise suitable excipients, such as pharmaceutically acceptable excipients including buffers, which are well known in the art.

### Methods of treating taxol-induced sensory neuropathy using agonist anti-trkC antibodies

The present invention encompasses treating, preventing delaying the development of a symptom of and/or palliating taxol-induced sensory neuropathy using agonist anti-trkC antibodies. The methods entail administering an effective amount of these antibodies to an individual in need thereof (various indications and aspects are described herein). An effective amount of the agonist anti-trkC antibody may be administered with or without other therapeutic agents. In some embodiments, the individual is human. However, the methods described are also applicable to the veterinary context (*e.g*. non-human mammal, such as dogs, cats, cattle, horses).

Methods of assessing taxol-induced sensory neuropathy and treatment thereof are known in the art and described herein.

In one aspect, the invention relates to treating a taxol-induced sensory neuropathy in an individual comprising administering an effective amount of agonist anti-trkC antibody. In yet another aspect, the invention relates to enhancing maintenance and/or regeneration of peripheral neurons in an individual having taxol-induced sensory neuropathy. The treatment of cancer with taxol may be enhanced as described herein, by administration of taxol in conjunction with an agonist anti-trkC antibody. Bone marrow transplantation may be enhanced as described herein, by administration of taxol in conjunction with an agonist anti-trkC antibody. In some embodiments, the individual is an individual at risk for taxol-induced neuropathy.

As is evident, agonist anti-trkC antibody can be administered before, during, or after treatment with taxol, or can be delivered before initiating course of taxol therapy; during a course of taxol therapy, and/or after cessation of a course of taxol therapy. Administration can be before onset of neuropathy. In some embodiments, the individual is undergoing treatment with taxol. In other embodiments, the individual is undergoing treatment with taxol and another agent (such as cis-platinum). In still other embodiments, the individual has had prior taxol treatment.

Taxol is approved for and commonly used for treating various malignancies, including Kaposi's sarcoma and those of the breast, ovary, and lung. Taxol is also used to treat other malignancies, including those of the prostate, head and neck, and various hematological malignancies. Taxol is also given during bone marrow transplants. Accordingly, in one embodiment of the invention, the individual treated with agonist anti-trkC antibody has one or more of: breast cancer, lung cancer, ovarian cancer, Kaposi's sarcoma, prostate cancer, cancer of the head and/or neck, and hematological malignancies. In another embodiment, the individual treated with agonist anti-trkC antibody requires or has received a bone marrow transplant. In another embodiment, the individual has an indication (such as cancer) that is treatable with taxol, or has been treated with taxol.

### Agonist anti-trkC antibodies

Uses of the invention entail using anti-trkC antibodies that interact with trkC in a manner that activates trkC. An anti-trkC agonist antibody should exhibit any one or more of the following characteristics: (a) bind to trkC receptor; (b) bind to one or more epitopes of trkC receptor; (c) binds to trkC receptor and activate trkC biological activity(ies) or one or more downstream pathways mediated by trkC signaling function(s); (d) bind to trkC receptor and treat, prevent, reverse, or ameliorate one or more symptoms of taxol-induced sensory neuropathy; (e) promote trkC receptor dimerization; (f) bind to trkC receptor, and treat, prevent, reverse, or ameliorate pain (including allodynia and/or hyperalgesia) associated with taxol-induced sensory neuropathy; (g) increase trkC receptor activation; (h) display favorable pharmacokinetic and bioavailability properties; (i) promote the development, survival, function, maintenance and/or regeneration of cells; (j) enhance taxol treatment of cancer; (k) enhance taxol treatment of bone marrow transplant.

Agonist anti-trkC antibodies are known in the art. *See* PCT Publication No. WO 01/98361; Urfer et al. J. Biol. Chem. (1998) 273:5829-5840. In some embodiments, the anti-trkC agonist antibody is a humanized mouse anti-trkC agonist antibody termed antibody "A5", which comprises the human heavy chain IgG2a constant region containing the following mutations: A330P331 to S330S331 (amino acid numbering with reference to the wildtype IgG2a sequence; *see* Eur. J. Immunol. (1999) 29:2613-2624); the human light chain kappa constant region; and the heavy chain and light chain variable regions shown in Tables 1 and 2.

Table 1: **A5 heavy chain variable region.** Kabat CDRs are shown as *underlined italics;* Chothia CDRs are shown as **bold**.

**Table 2: A5 light chain variable region.** Kabat CDRs are shown as *underlined italics*; Chothia CDRs are shown as **bold**.

The following polynucleotides encoding the heavy chain variable region or the light chain variable region of A5 were deposited with the American Type Culture Collection, 10801 University Boulevard, Manassas, Virginia, USA (ATCC):

| ***Material*** | | ATCC Accession No. | Date of Deposit |
|---|---|---|---|
| Eb.pur.2256.A5 | A5 light chain | PTA-5682 | December 5, 2003 |
| Db.2256.A5 | A5 heavy chain | PTA-5683 | December 5, 2003 |

Vector Eb.pur.2256.A5 is a polynucleotide encoding the A5 light chain variable region; and vector Db.2256.A5 is a polynucleotide encoding the A5 heavy chain variable region.

In other embodiments, the anti-trkC agonist antibody comprises one or more CDR(s) of antibody A5 (such as one, two, three, four, five, or, in some embodiments, all six CDR(s) from A5). Determination of CDR regions is well within the skill of the art. There are several techniques for determining CDRs: (1) an approach based on cross-species sequence variability (*i.e.,* Kabat et al. Sequences of Proteins of Immunological Interest, (5th ed., 1991, National Institutes of Health, Bethesda MD)); (2) an approach based on crystallographic studies of antigen-antibody complexes (Al-lazikani et al (1997) J. Molec. Biol. 273:927-948)). Identification of CDRs is well within the skill of the art. In some embodiments, the CDRs comprise the Kabat CDR. In other embodiments, the CDRs are the Chothia CDR. In still other embodiments, the CDR comprises both the Kabat and Chothia CDRs.

Antibodies can encompass monoclonal antibodies, polyclonal antibodies, antibody fragments (*e.g*., Fab, Fab', F(ab')₂, Fv, Fc, etc.), chimeric antibodies, single chain (ScFv), mutants thereof, fusion proteins comprising an antibody portion, and any other modified configuration of the immunoglobulin molecule that comprises an antigen recognition site of the required specificity. The antibodies may be murine, rat, human, or any other origin (including humanized antibodies). Thus, the agonist anti-trkC antibody may be a human antibody (such as antibody 6.4.1 (PCT Publication No. WO 01/98361)) or may be a humanized antibody (including humanized monoclonal antibody A5).

The agonist anti-trkC antibody may bind human trkC. The agonist anti-trkC antibody may also bind human and rodent trkC. In some embodiments, the agonist anti-trkC antibody may bind human and rat trkC. In some embodiments, the anti-trkC antibody may bind human and mouse trkC. In one embodiment, the antibody is an antibody that recognizes one or more epitopes on human trkC extracellular domain. In another embodiment, the antibody is a mouse or rat antibody that recognizes one or more epitopes on human trkC extracellular domain. In some embodiments, the antibody binds human trkC and does not significantly bind trkC from another mammalian species (in some embodiments, vertebrate species). In some embodiments, the antibody binds human trkC as well as one or more trkC from another mammalian species (in some embodiments, vertebrate species). In another embodiment, the antibody recognizes one or more epitopes on a trkC selected from one or more of: primate, canine, feline, equine, and bovine. In some embodiments, the antibody binds trkC and does not significantly cross-react (bind) with other neurotrophin receptors (such as the related neurotrophin receptors, trkA and/or trkB). In some embodiments, the antibody binds trkC, and further binds trkA and/or trkB.

The epitope(s) recognized by the trkC agonist antibody can be continuous or discontinuous. In some embodiments, the antibody binds essentially the same trkC epitope as an antibody selected from any one or more of the following: 6.1.2, 6.4.1, 2345, 2349, 2.5.1, 2344, 2248, 2250, 2253, and 2256. *See* PCT Publication No. WO 01/98361. Examples of epitopes to which an antibody may be directed include but are not limited to domain V and/or domain IV oftrkC. In another embodiment, the epitope includes one or more of the following residues: L284, E287, and N335 of human trkC. *See* Urfer et al. J. Biol. Chem. (1998) 273:5829-5840). In still other embodiments, the antibody comprises a modified constant region, such as a constant region that is immunologically inert, *e.g*., does not trigger a complement mediated lysis or does not stimulate antibody-dependent cell mediated cytotoxicity (ADCC). In other embodiments, the constant region is modified as described in Eur. J. Immunol. (1999) 29:2613-2624; PCT Application No. PCT/GB99/01441; and/or UK Patent application No. 9809951.8. In some embodiments, the constant region comprises the human heavy chain IgG2a constant region containing the following mutations: A330P331 to S330S331 (amino acid numbering with reference to the wildtype IgG2a sequence; *see* Eur. J. Immunol. (1999) 29:2613-2624).

The binding affinity of anti-trkC agonist antibody to trkC may be any of about 500 nM, 400 nM, 300 nM, 200 nM, 100 nM, about 50 nM, about 10 nM, about 1 nM, about 500 pM, about 100 pM, or about 50 pM to any of about 2 pM, about 5 pM, about 10 pM, about 15 pM, about 20 pM, or about 40 pM. In some embodiments, the binding affinity is any of about 100 nM, about 50 nM, about 10 nM, about 1 nM, about 500 pM, about 100 pM, or about 50 pM, or less than about 50 pM. In some embodiments, the binding affinity is less than any of about 100 nM, about 50 nM, about 10 nM, about 1 nM, about 500 pM, about 100 pM, or about 50 pM. In still other embodiments, the binding affinity is about 2 pM, about 5 pM, about 10 pM, about 15 pM, about 20 pM, about 40 pM, or greater than about 40 pM. As is well known in the art, binding affinity can be expressed as K_{D}, or dissociation constant, and an increased binding affinity corresponds to a decreased K_{D}. The binding affinity of mouse anti-trkC agonist monoclonal antibody 2256 to human trkC is about 40 nM, as assessed using BIAcore analysis, and the binding affinity of humanized anti-trkC agonist antibody A5 (described herein) to human trkC is about 0.28 nM, as assessed using BIAcore analysis.

One way of determining binding affinity of antibodies to trkC is by measuring binding affinity of monofunctional Fab fragments of the antibody. To obtain monofunctional Fab fragments, an antibody (for example, IgG) can be cleaved with papain or expressed recombinantly. The affinity of an anti-trkC Fab fragment of an antibody can be determined by surface plasmon resonance (B1Acore3000™ surface plasmon resonance (SPR) system, BIAcore, INC, Piscaway NJ). CM5 chips can be activated with N-ethyl-N'-(3-dimethylaminopropyl)-carbodiinide hydrochloride (EDC) and N-hydroxysuccinimide (NHS) according to the supplier's instructions. Human trkC-Fc fusion protein ("htrkC") (or any other trkC, such as rat trkC) can be diluted into 10 mM sodium acetate pH 5.0 and injected over the activated chip at a concentration of 0.0005 mg/mL. Using variable flow time across the individual chip channels, two ranges of antigen density can be achieved: 200-400 response units (RU) for detailed kinetic studies and 500-1000 RU for screening assays. The chip can be blocked with ethanolamine. Regeneration studies have shown that a mixture of Pierce elution buffer (Product No. 21004, Pierce Biotechnology, Rockford, IL) and 4 M NaCl (2:1) effectively removes the bound Fab while keeping the activity of htrkC on the chip for over 200 injections. HBS-EP buffer (0.01M HEPES, pH 7.4, 0.15 NaCl, 3mM EDTA, 0.005% Surfactant P29) is used as running buffer for the BIAcore assays. Serial dilutions (0.1-10x estimated K_{D}) of purified Fab samples are injected for 1 min at 100 µL/min and dissociation times of up to 2h are allowed. The concentrations of the Fab proteins are determined by ELISA and/or SDS-PAGE electrophoresis using a Fab of known concentration (as determined by amino acid analysis) as a standard. Kinetic association rates (kₒₙ) and dissociation rates (k_{off}) are obtained simultaneously by fitting the data to a 1:1 Langmuir binding model (Karlsson, R. Roos, H. Fagerstam, L. Petersson, B. (1994). Methods Enzymology 6:99-110) using the BIAevaluation program. Equilibrium dissociation constant (K_{D}) values are calculated as k_{off}/kₒₙ.

In another aspect, antibodies (e.g., human, humanized, mouse, chimeric) that can activate human trkC receptor may be made by using immunogens which express one or more extracellular domains of trkC. One example of an immunogen is cells with high expression of trkC, which can be obtained as described herein. Another example of an immunogen that can be used is a soluble protein (such as a trkC immunoadhesin) which contains the extracellular domain or a portion of the extracellular domain of trkC receptor.

The route and schedule of immunization of the host animal are generally in keeping with established and conventional techniques for antibody stimulation and production, as further described herein. General techniques for production of human and mouse antibodies are known in the art and are described herein.

It is contemplated that any mammalian subject including humans or antibody producing cells therefrom can be manipulated to serve as the basis for production of mammalian, including human, hybridoma cell lines. Typically, the host animal is inoculated intraperitoneally with an amount of immunogen, including as described herein.

Hybridomas can be prepared from the lymphocytes and immortalized myeloma cells using the general somatic cell hybridization technique of Kohler, B. and Milstein, C. (1975) Nature 256:495-497 or as modified by Buck, D. W. et al., (1982) In Vitro, 18:377-381. Available myeloma lines, including but not limited to X63-Ag8.653 and those from the Salk Institute, Cell Distribution Center, San Diego, Calif., USA, may be used in the hybridization. Generally, the technique involves fusing myeloma cells and lymphoid cells using a fusogen such as polyethylene glycol, or by electrical means well known to those skilled in the art. After the fusion, the cells are separated from the fusion medium and grown in a selective growth medium, such as hypoxanthine-aminopterin-thymidine (HAT) medium, to eliminate unhybridized parent cells. Any of the media described herein, supplemented with or without serum, can be used for culturing hybridomas that secrete monoclonal antibodies. As another alternative to the cell fusion technique, EBV immortalized B cells may be used to produce the anti-trkC monoclonal antibodies of the subject invention. The hybridomas are expanded and subcloned, if desired, and supernatants are assayed for anti-immunogen activity by conventional immunoassay procedures (*e.g*., radioimmunoassay, enzyme immunoassay, or fluorescence immunoassay).

Hybridomas that may be used as source of antibodies encompass all derivatives, progeny cells of the parent hybridomas that produce monoclonal antibodies specific for trkC, or a portion thereof.

Hybridomas that produce such antibodies may be grown in vitro or in vivo using known procedures. The monoclonal antibodies may be isolated from the culture media or body fluids, by conventional immunoglobulin purification procedures such as ammonium sulfate precipitation, gel electrophoresis, dialysis, chromatography, and ultrafiltration, if desired. Undesired activity if present, can be removed, for example, by running the preparation over adsorbents made of the immunogen attached to a solid phase and eluting or releasing the desired antibodies off the immunogen. Immunization of a host animal with a human or other species of trkC receptor, or a fragment of the human or other species of trkC receptor, or a human or other species of trkC receptor or a fragment containing the target amino acid sequence conjugated to a protein that is immunogenic in the species to be immunized, *e.g*., keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glytaradehyde, succinic anhydride, SOCl2, or R1N=C=NR, where R and R1 are different alkyl groups can yield a population of antibodies (*e.g*., monoclonal antibodies). Another example of an immunogen is cells with high expression of trkC, which can be obtained from recombinant means, or by isolating or enriching cells from a natural source that express a high level of trkC. These cells may be of human or other animal origin, and may be used as an immunogen as directly isolated, or may be processed in such that immunogenicity is increased, or trkC expression (of a fragment of trkC) is increased or enriched. Such processing includes, but is not limited to, treatment of the cells or fragments thereof with agents designed to increase their stability or immunogenicity, such as, *e.g*., formaldehyde, glutaraldehyde, ethanol, acetone, and/or various acids. Further, either before or after such treatment the cells may be processed in order to enrich for the desired immunogen, in this case trkC or fragment thereof. These processing steps can include membrane fractionation techniques, which are well known in the art.

If desired, the anti-trkC antibody (monoclonal or polyclonal) of interest may be sequenced and the polynucleotide sequence may then be cloned into a vector for expression or propagation. The sequence encoding the antibody of interest may be maintained in a vector in a host cell and the host cell can then be expanded and frozen for future use. As an alternative, the polynucleotide sequence may be used for genetic manipulation to "humanize" the antibody or to improve the affinity, or other characteristics of the antibody. For example, the constant region may be engineered to more resemble human constant regions to avoid immune response if the antibody is used in clinical trials and treatments in humans. It may be desirable to genetically manipulate the antibody sequence to obtain greater affinity to trkC receptor and greater efficacy in activating trkC receptor. It will be apparent to one of skill in the art that one or more polynucleotide changes can be made to the anti-trkC antibody and still maintain its binding ability to trkC extracellular domain or epitopes of trkC.

There are four general steps to humanize a monoclonal antibody. These are: (1) determining the nucleotide and predicted amino acid sequence of the starting antibody light and heavy variable domains (2) designing the humanized antibody, *i.e*., deciding which antibody framework region to use during the humanizing process (3) the actual humanizing methodologies/techniques and (4) the transfection and expression of the humanized antibody. *See,* for example, U.S. Patent Nos. 4,816,567; 5,807,715; 5,866,692; 6,331,415; 5,530,101; 5,693,761; 5,693,762; 5,585,089; 6,180,370; and 6,548,640. For example, the constant region may be engineered to more resemble human constant regions to avoid immune response if the antibody is used in clinical trials and treatments in humans. *See,* for example, U.S. Patent Nos. 5,997,867 and 5,866,692.

In the recombinant humanized antibodies, the Fcγ portion can be modified to avoid interaction with Fcγ receptor and the complement immune system. This type of modification was designed by Dr. Mike Clark from the Department of Pathology at Cambridge University, and techniques for preparation of such antibodies are described in PCT Publication No. WO 99/58572, published November 18, 1999.

A number of "humanized" antibody molecules comprising an antigen-binding site derived from a non-human immunoglobulin have been described, including chimeric antibodies having rodent or modified rodent V regions and their associated complementarity determining regions (CDRs) fused to human constant domains. *See*, for example, Winter et al. Nature 349:293-299 (1991), Lobuglio et al. Proc. Nat. Acad. Sci. USA 86:4220-4224 (1989), Shaw et al. J Immunol. 138:4534-4538 (1987), and Brown et al. Cancer Res. 47:3577-3583 (1987). Other references describe rodent CDRs grafted into a human supporting framework region (FR) prior to fusion with an appropriate human antibody constant domain. *See,* for example, Riechmann et al. Nature 332:323-327 (1988), Verhoeyen et al. Science 239:1534-1536 (1988), and Jones et al. Nature 321:522-525 (1986). Another reference describes rodent CDRs supported by recombinantly veneered rodent framework regions. *See,* for example, European Patent Publication No. 519,596. These "humanized" molecules are designed to minimize unwanted immunological response toward rodent anti-human antibody molecules which limits the duration and effectiveness of therapeutic applications of those moieties in human recipients. The antibody constant region can be engineered such that it is immunologically inert, *e.g*., does not trigger a complement mediated lysis or does not stimulate antibody-dependent cell mediated cytotoxicity (ADCC). In other embodiments, the constant region is modified as described in Eur. J. Immunol. (1999) 29:2613-2624; PCT Application No. PCT/GB99/01441; and/or UK Patent Application No. 9809951.8.
*See, e.g.* PCT/GB99/01441; UK patent Application No. 9809951.8. Other methods of humanizing antibodies that may also be utilized are disclosed by Daugherty et al., Nucl. Acids Res. 19:2471-2476 (1991) and in U.S. Patent Nos. 6,180,377; 6,054,297; 5,997,867; 5,866,692; 6,210,671; 6,350,861; and PCT Publication No. WO 01/27160.

In yet another alternative, fully human antibodies may be obtained by using commercially available mice that have been engineered to express specific human immunoglobulin proteins. Transgenic animals that are designed to produce a more desirable (e.g., fully human antibodies) or more robust immune response may also be used for generation of humanized or human antibodies. Examples of such technology are Xenomouse™ from Abgenix, Inc. (Fremont, CA) and HuMAb-Mouse® and TC Mouse™ from Medarex, Inc. (Princeton, NJ).

In an alternative, antibodies may be made recombinantly and expressed using any method known in the art. In another alternative, antibodies may be made recombinantly by phage display technology. *See,* for example, U.S. Patent Nos. 5,565,332; 5,580,717; 5,733,743 and 6,265,150; and Winter et al., Annu. Rev. Immunol. 12:433-455 (1994). Alternatively, the phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for review *see, e.g.,* Johnson, Kevin S. and Chiswell, David J., Current Opinion in Structural Biology 3, 564-571 (1993). Several sources of V-gene segments can be used for phage display. Clackson et al., Nature 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Mark et al., J. Mol. Biol. 222:581-597 (1991), or Griffith et al., EMBO J. 12:725-734 (1993). In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling." Marks, et al., Bio/Technol. 10:779-783 (1992)). In this method, the affinity of "primary" human antibodies obtained by phage display can be improved by sequentially replacing the heavy and light chain V region genes with repertoires of naturally occurring variants (repertoires) of V domain genes obtained from unimmunized donors. This technique allows the production of antibodies and antibody fragments with affinities in the pM-nM range. A strategy for making very large phage antibody repertoires (also known as "the mother-of-all libraries") has been described by Waterhouse et al., Nucl. Acids Res. 21:2265-2266 (1993). Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable regions capable of restoring a functional antigen-binding site, *i.e.,* the epitope governs (imprints) the choice of partner. When the process is repeated in order to replace the remaining rodent V domain, a human antibody is obtained *(see* PCT Publication No. WO 93/06213, published April 1, 1993). Unlike traditional humanization of rodent antibodies by CDR grafting, this technique provides completely human antibodies, which have no framework or CDR residues of rodent origin. It is apparent that although the above discussion pertains to humanized antibodies, the general principles discussed are applicable to customizing antibodies for use, for example, in dogs, cats, primates, equines and bovines.

The antibody may be a bispecific antibody, a monoclonal antibody that has binding specificities for at least two different antigens, can be prepared using the antibodies disclosed herein. Methods for making bispecific antibodies are known in the art (*see, e.g.,* Suresh et al., 1986, Methods in Enzymology 121:210). Traditionally, the recombinant production of bispecific antibodies was based on the coexpression of two immunoglobulin heavy chain-light chain pairs, with the two heavy chains having different specificities (Millstein and Cuello, 1983, Nature 305, 537-539).

According to one approach to making bispecific antibodies, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2 and CH3 regions. It is preferred to have the first heavy chain constant region (CH1), containing the site necessary for light chain binding, present in at least one of the fusions. DNAs encoding the immunoglobulin heavy chain fusions and, if desired, the immunoglobulin light chain, are inserted into separate expression vectors, and are cotransfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one approach, the bispecific antibodies are composed of a hybrid immunoglobulin heavy chain with a first binding specificity in one arm, and a hybrid immunoglobulin heavy chain light chain pair (providing a second binding specificity) in the other arm. This asymmetric structure, with an immunoglobulin light chain in only one half of the bispecific molecule, facilitates the separation of the desired bispecific compound from unwanted immunoglobulin chain combinations. This approach is described in PCT Publication No. WO 94/04690, published March 3,1994.

Heteroconjugate antibodies, comprising two covalently joined antibodies, are also within the scope of the invention. Such antibodies have been used to target immune system cells to unwanted cells (U.S. Patent No. 4,676,980), and for treatment of HIV infection (PCT Publication Nos. WO 91/00360 and WO 92/200373; and EP 03089). Heteroconjugate antibodies may be made using any convenient cross-linking methods. Suitable cross-linking agents and techniques are well known in the art, and are described in U.S. Patent No. 4,676,980.

Antibodies may be made recombinantly by first isolating the antibodies made from host animals, obtaining the gene sequence, and using the gene sequence to express the antibody recombinantly in host cells (*e.g*., CHO cells). Another method that may be employed is to express the antibody sequence in plants (*e.g*., tobacco), transgenic milk, or in other organisms. Methods for expressing antibodies recombinantly in plants or milk have been disclosed. *See,* for example, Peeters et al. (2001) Vaccine 19:2756; Lonberg, N. and D. Huszar (1995) Int.Rev.Immunol 13:65; and Pollock et al. (1999) J Immunol Methods 231:147. Methods for making derivatives of antibodies, *e.g*., humanized, single chain, etc. are known in the art.

Chimeric or hybrid antibodies also may be prepared *in vitro* using known methods of synthetic protein chemistry, including those involving cross-linking agents. For example, immunotoxins may be constructed using a disulfide exchange reaction or by forming a thioether bond. Examples of suitable reagents for this purpose include iminothiolate and methyl-4-mercaptobutyrimidate.

Single chain Fv fragments may also be produced, such as described in Iliades et al., 1997, FEBS Letters, 409:437-441. Coupling of such single chain fragments using various linkers is described in Kortt et al., 1997, Protein Engineering, 10:423-433. A variety of techniques for the recombinant production and manipulation of antibodies are well known in the art.

Antibodies may be modified as described in PCT Publication No. WO 99/58572, published November 18, 1999. These antibodies comprise, in addition to a binding domain directed at the target molecule, an effector domain having an amino acid sequence substantially homologous to all or part of a constant domain of a human immunoglobulin heavy chain. These antibodies are capable of binding the target molecule without triggering significant complement dependent lysis, or cell-mediated destruction of the target. Preferably, the effector domain is capable of specifically binding FcRn and/or FcγRIIb. These are typically based on chimeric domains derived from two or more human immunoglobulin heavy chain C_{H}2 domains. Antibodies modified in this manner are preferred for use in chronic antibody therapy, to avoid inflammatory and other adverse reactions to conventional antibody therapy.

The antibodies made either by immunization of a host animal or recombinantly should exhibit any one or more of the following characteristics: (a) bind to trkC receptor; (b) bind to one or more epitopes of trkC receptor; (c) binds to trkC receptor and activate trkC biological activity(ies) or one or more downstream pathways mediated by trkC signaling function(s); (d) bind to trkC receptor and treat, prevent, reverse, or ameliorate one or more symptoms of taxol-induced sensory neuropathy; (e) promote trkC receptor dimerization; (f) bind to trkC receptor, and treat, prevent, reverse, or ameliorate pain (including allodynia and/or hyperalgesia) associated with taxol-induced sensory neuropathy; (g) increase trkC receptor activation; (h) display favorable pharmacokinetic and bioavailability properties; (i) promote the development, survival, function, maintenance and/or regeneration of cells; (j) enhance taxol treatment of cancer; (k) enhance taxol treatment of bone marrow transplant.

Immunoassays and flow cytometry sorting techniques such as fluorescence activated cell sorting (FACS) can also be employed to isolate antibodies that are specific for trkC.

The antibodies can be bound to many different carriers. Carriers can be active and/or inert. Examples of well-known carriers include polypropylene, polystyrene, polyethylene, dextran, nylon, amylases, glass, natural and modified celluloses, polyacrylamides, agaroses and magnetite. The nature of the carrier can be either soluble or insoluble for purposes of the invention. Those skilled in the art will know of other suitable carriers for binding antibodies, or will be able to ascertain such, using routine experimentation.

DNA encoding agonist anti-trkC antibodies may be sequenced, as is known in the art. *See* PCT Publication No. WO 01/98361. Generally, the monoclonal antibody is readily isolated and sequenced using conventional procedures (*e.g*., by using oligonucleotide probes that are capable of binding specifically to genes encoding the heavy and light chains of the monoclonal antibodies). The hybridoma cells serve as a preferred source of such cDNA. Once isolated, the DNA may be placed into expression vectors (such as expression vectors disclosed in PCT Publication No. WO 87/04462), which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. *See, e.g.,* PCT Publication No. WO 87/04462. The DNA also may be modified, for example, by substituting the coding sequence for human heavy and light chain constant domains in place of the homologous murine sequences, Morrison et al., Proc. Nat. Acad. Sci. 81: 6851 (1984), or by covalently joining to the immunoglobulin coding sequence all or part of the coding sequence for a non-immunoglobulin polypeptide. In that manner, "chimeric" or "hybrid" antibodies are prepared that have the binding specificity of an anti-trkC monoclonal antibody herein. The DNA encoding the agonist anti-trkC antibody (such as an antigen binding fragment thereof) may also be used for delivery and expression of agonist anti-trkC antibody in a desired cell, as described here. DNA delivery techniques are further described herein.

Anti-trkC antibodies may be characterized using methods well-known in the art. For example, one method is to identify the epitope to which it binds, including soling the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, New York, 1999. In an additional example, epitope mapping can be used to determine the sequence to which an anti-trkC antibody binds. Epitope mapping is commercially available from various sources, for example, Pepscan Systems (Edelhertweg 15, 8219 PH Lelystad, The Netherlands). The epitope can be a linear epitope, *i.e.,* contained in a single stretch of amino acids, or a conformational epitope formed by a three-dimensional interaction of amino acids that may not necessarily be contained in a single stretch. Peptides of varying lengths (*e.g*., at least 4-6 amino acids long) can be isolated or synthesized (*e.g*., recombinantly) and used for binding assays with an anti-trkC antibody. In another example, the epitope to which the anti-trkC antibody binds can be determined in a systematic screening by using overlapping peptides derived from the trkC extracellular sequence and determining binding by the anti-trkC antibody. According to the gene fragment expression assays, the open reading frame encoding trkC is fragmented either randomly or by specific genetic constructions and the reactivity of the expressed fragments of trkC with the antibody to be tested is determined. The gene fragments may, for example, be produced by PCR and then transcribed and translated into protein in vitro, in the presence of radioactive amino acids. The binding of the antibody to the radioactively labeled trkC fragments is then determined by immunoprecipitation and gel electrophoresis. Certain epitopes can also be identified by using large libraries of random peptide sequences displayed on the surface of phage particles (phage libraries).

Yet another method which can be used to characterize an anti-trkC antibody is to use competition assays with other antibodies known to bind to the same antigen, *i.e.,* trkC extracellular domain to determine if the anti-trkC antibody binds to the same epitope as other antibodies. Competition assays are well known to those of skill in the art. Examples of antibodies useful in competition assays include the following: antibodies 6.1.2, 6.4.1, 2345, 2349, 2.5.1, 2344, 2248, 2250, 2253, and 2256. *See* PCT Publication No. WO 01/98361

Epitope mapping can also be performed using domain swap mutants as described in PCT Publication No. WO 01/98361. Generally, this approach is useful for anti-trkC antibodies that do not significantly cross-react with trkA or trkB. Domain-swap mutants of trkC can be made by replacing extracellular domains of trkC with the corresponding domains from trkB or trkA. The binding of each agonist anti-trkC antibody to various domain-swap mutants can be evaluated and compared to its binding to wild type (native) trkC using ELISA or other method known in the art. In another approach, alanine scanning can be performed. Individual residues of the antigen, the trkC receptor, are systematically mutated to another amino acid (usually alanine) and the effect of the changes is assessed by testing the ability of the modified trkC to bind to antibody using ELISA or other methods known in the art.

### Identification of agonist anti-trkC antibodies

Agonist antibodies may be identified using art-recognized methods, including one or more of the following methods. For example, the kinase receptor activation (KIRA) assay described in U. S. Patent Nos. 5,766,863 and 5,891,650 may be used. This ELISA-type assay is suitable for qualitative or quantitative measurement of kinase activation by measuring the autophosphorylation of the kinase domain of a receptor protein tyrosine kinase (rPTK, *e.g*. trk receptor), as well as for identification and characterization of potential agonist or antagonists of a selected rPTK. The first stage of the assay involves phosphorylation of the kinase domain of a kinase receptor, in the present case a trkC receptor, wherein the receptor is present in the cell membrane of a eukaryotic cell. The receptor may be an endogenous receptor or nucleic acid encoding the receptor, or a receptor construct, may be transformed into the cell. Typically, a first solid phase (*e.g*., a well of a first assay plate) is coated with a substantially homogeneous population of such cells (usually a mammalian cell line) so that the cells adhere to the solid phase. Often, the cells are adherent and thereby adhere naturally to the first solid phase. If a "receptor construct" is used, it usually comprises a fusion of a kinase receptor and a flag polypeptide. The flag polypeptide is recognized by the capture agent, often a capture antibody, in the ELISA part of the assay. An analyte, such as a candidate agonist, is then added to the wells having the adherent cells, such that the tyrosine kinase receptor (*e.g*. trkC receptor) is exposed to (or contacted with) the analyte. This assay enables identification of agonist ligands for the tyrosine kinase receptor of interest (*e.g*. trkC). Following exposure to the analyte, the adhering calls are solubilized using a lysis buffer (which has a solubilizing detergent therein) and gentle agitation, thereby releasing cell lysate which can be subjected to the ELISA part of the assay directly, without the need for concentration or clarification of the cell lysate.

The cell lysate thus prepared is then ready to be subjected to the ELISA stage of the assay. As a first step in the ELISA stage, a second solid phase (usually a well of an ELISA microtiter plate) is coated with a capture agent (often a capture antibody) that binds specifically to the tyrosine kinase receptor, or, in the case of a receptor construct, to the flag polypeptide. Coating of the second solid phase is carried out so that the capture agent adheres to the second solid phase. The capture agent is generally a monoclonal antibody, but, as is described in the examples herein, polyclonal antibodies or other agents may also be used. The cell lysate obtained is then exposed to, or contacted with, the adhering capture agent so that the receptor or receptor construct adheres to (or is captured in) the second solid phase. A washing step is then carried out, so as to remove unbound cell lysate, leaving the captured receptor or receptor construct. The adhering or captured receptor or receptor construct is then exposed to, or contacted with, an anti-phosphotyrosine antibody which identifies phosphorylated tyrosine residues in the tyrosine kinase receptor. In the preferred embodiment, the anti phosphotyrosine antibody is conjugated (directly or indirectly) to an enzyme which catalyses a color change of a non-radioactive color reagent. Accordingly, phosphorylation of the receptor can be measured by a subsequent color change of the reagent. The enzyme can be bound to the anti-phosphotyrosine antibody directly, or a conjugating molecule (*e.g*., biotin) can be conjugated to the anti-phosphotyrosine antibody and the enzyme can be subsequently bound to the anti-phosphotyrosine antibody via the conjugating molecule. Finally, binding of the anti-phosphotyrosine antibody to the captured receptor or receptor construct is measured, *e.g*., by a color change in the color reagent.

Following initial identification, the agonist activity of a candidate antibody can be further confirmed and refined by bioassays, known to test the targeted biological activities. For example, the ability of anti-trkC monoclonal antibodies to agonize trkC can be tested in the PC12 neurite outgrowth assay using PC12 cells transfected with full-length human trkC (Urfer et al., Biochem. 36: 4775-4781 (1997); Tsoulfas et al., Neuron 10: 975-990 (1993)). This assay measures the outgrowth of neurite processes by rat pheocytochroma cells (PC12) in response to stimulation by appropriate ligands. These cells express endogenous trkA and are therefore responsive to NGF. However, they do not express endogenous trkC and are therefore transfected with trkC expression construct in order to elicit response to trkC agonists. After incubating the transfected cells with anti-trkC antibodies, neurite outgrowth is measured, and *e.g*., cells with neurites exceeding 2 times the diameter of the cell are counted. Anti-trkC antibodies that stimulate neurite outgrowth in transfected PC12 cells demonstrate trkC agonist activity.

The activation of trkC may also be determined by using various specific neurons at specific stages of embryonic development. Appropriately selected neurons can be dependent on trkC activation for survival, and so it is possible to determine the activation of trkC by following the survival of these neurons in vitro. Addition of candidate antibodies to primary cultures of appropriate neurons will lead to survival of these neurons for a period of at least several days if the candidate antibodies activate trkC. This allows the determination of the ability of the candidate antibody to activate trkC. In one example of this type of assay, the trigeminal ganglion from an E11 mouse embryo is dissected, dissociated and the resultant neurons are plated in a tissue culture dish at low density. The candidate antibodies are then added to the media and the plates incubated for 24-48 hours. After this time, survival of the neurons is assessed by any of a variety of methods. Samples which received an agonist anti-trkC antibody will typically display an increased survival rate over samples which receive a control antibody, and this allows the determination of the presence of an agonist anti-trkC antibody. *See, e.g.,* Buchman et al (1993) Development, 118(3):989-1001.

Agonist antibodies may be identified by their ability to activate downstream signaling in a variety of cell types that express trkC, either naturally or after transfection of DNA encoding trkC. This trkC may be human or other mammalian (such a rodent or primate) trkC. The downstream signaling cascade may be detected by changes to a variety of biochemical or physiological parameters of the trkC expressing cell, such as the level of protein expression or of protein phosphorylation of proteins or changes to the metabolic or growth state of the cell (including neuronal survival and/or neurite outgrowth, as described herein). Methods of detecting relevant biochemical or physiological parameters are known in the art.

### Administration of agonist anti-trkC antibodies

Various formulations of agonist anti-trkC antibodies may be used for administration. In some embodiments, an agonist anti-trkC antibody may be administered neat. In some embodiments, an agonist anti-trkC antibody is administered in a composition comprising a pharmaceutically acceptable excipient. Pharmaceutically acceptable excipients are known in the art, and are relatively inert substances that facilitate administration of a pharmacologically effective substance. For example, an excipient can give form or consistency, or act as a diluent. Suitable excipients include but are not limited to stabilizing agents, wetting and emulsifying agents, salts for varying osmolarity, encapsulating agents, buffers, and skin penetration enhancers. Excipients as well as formulations for parenteral and nonparenteral drug delivery are set forth in Remington, The Science and Practice of Pharmacy 20th Ed. Mack Publishing (2000).

Agonist anti-trkC antibodies can be formulated for administration by injection (e.g., intraperitoneally, intravenously, subcutaneously, intramuscularly, etc.). Accordingly, the antibodies may be combined with pharmaceutically acceptable vehicles such as saline, Ringer's solution, dextrose solution, and the like. The particular dosage regimen, *i.e.,* dose, timing and repetition, will depend on the particular individual and that individual's medical history. Generally, a dose of less than about 1 ug/kg body weigh, at least about 1 ug/kg body weight; at least about 2 ug/kg body weight, at least about 5 ug/kg body weight, at least about 10 ug/kg body weight, at least about 20 ug/kg body weight, at least about 50 ug/kg body weight, at least about 100 ug/kg body weight, at least about 200 ug/kg body weight, at least about 500 ug/kg body weight, at least about 1 mg/kg, body weight, at least about 2 mg/kg body weight, at least about 5 mg /kg body weight, at least about 10 mg/kg body weight, at least about 30 mg/kg body weight, or more (such as about 50 mg/kg, about 100 mg/kg, about 200 mg/kg or about 500 mg/kg) is administered.

Empirical considerations, such as the half-life, generally will contribute to the determination of the dosage. Antibodies which are compatible with the human immune system, such as humanized antibodies or fully human antibodies, may be used to prolong half-life of the antibody and to prevent the antibody being attacked by the host's immune system. Frequency of administration may be determined and adjusted over the course of therapy, and is generally, but not necessarily, based on maintaining an effective concentration of agonist anti-trkC antibody in the patient and suppression and/or amelioration and/or delay of one or more symptoms of taxol sensory neuropathy. Alternatively, sustained continuous release formulations of agonist anti-trkC antibodies may be appropriate. Various formulations and devices for achieving sustained release are known in the art. Administration of an agonist anti-trkC antibody in accordance with the method in the present invention can be continuous or intermittent, depending, for example, upon the recipient's physiological condition, whether the purpose of the administration is therapeutic or prophylactic, and other factors known to skilled practitioners. The administration of an agonist anti-trkC antibody may be essentially continuous over a preselected period of time or may be in a series of spaced dose, *e.g*., either before; during; or after developing symptoms of taxol-induced sensory neuropathy; before, and during; before and after; during and after; and/or before, during, and after developing symptoms of taxol-induced sensory neuropathy.

Generally, for administration of agonist anti-trkC antibodies, an initial candidate dosage can be about 2 mg/kg. For the purpose of the present invention, a typical daily dosage might range from about 30µg/kg to 100 mg/kg or more, depending on the factors mentioned above. For repeated administrations over several days or longer, depending on the condition, the treatment is sustained until a desired suppression of disease symptoms occurs or until sufficient therapeutic levels are achieved to treat or prevent taxol-induced sensory neuropathy. An exemplary dosing regimen comprises administering an initial dose of about 2 mg/kg, followed by a weekly maintenance dose of about 1 mg/kg of the trkC agonist antibody, or followed by a maintenance dose of about 1 mg/kg every other week.

In one embodiment, dosages for an antibody may be determined empirically in individuals who have been given one or more administration(s) of an agonist anti-trkC antibody that activates trkC receptor to treat a taxol-induced sensory neuropathy. Individuals are given incremental dosages of an agonist anti-trkC antibody. To assess efficacy of agonist anti-trkC antibodies, an indicator of taxol-induced sensory neuropathy disease state can be followed as described herein.

Other formulations include suitable delivery forms known in the art including, but not limited to, carriers such as liposome. *See,* for example, Mahato et al. (1997) Pharm. Res. 14:853-859. Liposomal preparations include, but are not limited to, cytofectins, multilamellar vesicles and unilamellar vesicles.

The formulations to be used for *in vivo* administration must be sterile. This is readily accomplished by, for example, filtration through sterile filtration membranes. Therapeutic agonist anti-trkC antibody compositions are generally placed into a container having a sterile access port, for example, an intravenous solution bag or vial having a stopper pierceable by a hypodermic injection needle.

The agonist anti-trkC antibody is administered to a individual in accord with known methods, such as intravenous administration, *e.g*., as a bolus or by continuous infusion over a period of time, by intramuscular, intraperitoneal, subcutaneous, oral, intrathecal, or topical routes. Agonist anti-trkC antibody can also be administered by inhalation. Commercially available nebulizers for liquid formulations, including jet nebulizers and ultrasonic nebulizers are useful for administration. Liquid formulations can be directly nebulized and lyophilized powder can be nebulized after reconstitution. Alternatively, agonist anti-trkC antibody can be aerosolized using a fluorocarbon formulation and a metered dose inhaler, or inhaled as a lyophilized and milled powder.

In some embodiments, more than one antibody may be present. The antibodies can be the same or different from each other. In some embodiments, at least one, at least two, at least three, at least four, at least five different trkC agonist antibodies are present. Preferably those antibodies have complementary activities that do not adversely affect each other.

A polynucleotide encoding an agonist anti-trkC antibody (such as an antigen binding fragment thereof) may also be used for delivery and expression of agonist anti-trkC antibody in a desired cell. It is apparent that an expression vector can be used to direct expression of an agonist anti-trkC antibody. The expression vector can be administered intraperitoneally, intravenously, intramuscularly, subcutaneously, intrathecally, intraventricularly, orally, enterally, parenterally, intranasally, dermally, or by inhalation. For example, administration of expression vectors includes local or systemic administration, including injection, oral administration, particle gun or catheterized administration, and topical administration. One skilled in the art is familiar with administration of expression vectors to obtain expression of an exogenous protein in vivo. *See, e.g.,* U.S. Patent Nos. 6,436,908; 6,413,942; and 6,376,471.

Targeted delivery of therapeutic compositions comprising a polynucleotide encoding an agonist anti-trkC antibody can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. (USA) (1990) 87:3655; Wu et al., J. Biol. Chem. (1991) 266:338. Therapeutic compositions containing a polynucleotide are administered in a range of about 100 ng to about 200 mg of DNA for local administration in a gene therapy protocol. Concentration ranges of about 500 ng to about 50 mg, about 1 µg to about 2 mg, about 5 µg to about 500 µg, and about 20 µg to about 100 µg of DNA can also be used during a gene therapy protocol. The therapeutic polynucleotides and polypeptides of the present invention can be delivered using gene delivery vehicles. The gene delivery vehicle can be of viral or non-viral origin (*see* generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (*see, e.g.,* PCT Publication Nos. WO 90/07936; WO 94/03622; WO 93/25698; WO 93/25234; WO 93/11230; WO 93/10218; WO 91/02805; U.S. Patent Nos. 5, 219,740; 4,777,127; GB Patent No. 2,200,651; and EP 0 345 242), alphavirus-based vectors (*e.g*., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (*see, e.g.,* PCT Publication Nos. WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (*see, e.g.,* Curiel, Hum. Gene Ther. (1992) 3:147); ligand-linked DNA (*see, e.g.,* Wu, J. Biol. Chem. (1989) 264:16985); eukaryotic cell delivery vehicles cells (*see, e.g.,* U.S. Patent No. 5,814,482; PCT Publication Nos. WO 95/07994; WO 96/17072; WO 95/30763; and WO 97/42338) and nucleic charge neutralization or fusion with cell membranes. Naked DNA can also be employed. Exemplary naked DNA introduction methods are described in PCT Publication No. WO 90/11092 and U.S. Patent No. 5,580,859. Liposomes that can act as gene delivery vehicles are described in U.S. Patent No. 5,422,120; PCT Publication Nos. WO 95/13796; WO 94/23697; WO 91/14445; and EP 0 524 968. Additional approaches are described in Philip, Mol. Cell Biol. (1994) 14:2411, and in Woffendin, Proc. Natl. Acad. Sci. (1994) 91:1581.

Taxol (including other taxanes) may be administered either alone, or in conjunction with other drugs. Most commonly, taxol is delivered in a formulation comprising ethanol and cremophor EL™, which is diluted into an aqueous salt solution for treatment. Taxol may also be formulated in various other configurations, such as in emulsions. Taxol is commonly administered in conjunction with radiotherapy and/or other chemotherapeutic drugs, including, but not limited to, various platinum containing compounds (cis-platinum, carboplatinum, oxaliplatin), ifosfamide, 5-fluorouracil, doxorubicin, epirubicin, cyclophosphamide, gemcitabine, capecitabine, exisulind, topotecan, etoposide, vinca alkaloids (vincristine, vinblatin, and vinorelbine), and other chemotherapeutic drugs known in the art. Taxol is also administered in conjunction with drugs designed to counteract or treat the adverse side effects of taxol and/or other chemotherapeutic agents that are administered in combination with taxol. For example, drugs such as erythropoietin (Epoiten, Darbopoiten), G-CSF, and GM-CSF) can be , administered in conjunction with taxol to treat the hematological effects of chemotherapeutic agents. As another example, drugs such as phenothiazines (Compazine), Zofran, and Anzemet can be administered in conjunction with taxol to treat the nausea which often accompanies the use of chemotherapeutic agents. Patients can be premedicated before taxol treatment

Taxol is approved for and commonly used for treating various malignancies, including Kaposi's sarcoma and those of the breast, ovary, and lung. Taxol is also used to treat other malignancies, including those of the prostate, head and neck, and various hematological malignancies. Taxol is also given during bone marrow transplants, *e.g*., to mobilize stem cells prior to bone marrow treatment. Representative dose regimens of taxol include: 135 mg/m² or 175 mg/m² administered intravenously over 3 hours every three weeks (for ovarian carcinoma); 175 mg/m² administered intravenously over 3 hours every three weeks (for breast carcinoma); 135 mg/m² administered intravenously over 24 hours (for non-small cell lung carcinoma); 135 mg/m² administered intravenously over 3 hours every three weeks or 100 mg/m² administered intravenously over 3 hours every two weeks (Kaposi's sarcoma). *See also* Taxol Prescribing Information (product insert), Bristol Meyers Squibb (1998) (available at http://www.taxol.com/txpi.html). In other embodiments, taxol is administered at 775 mg/m², 475mg/m², 200 mg/m², and/or 350 mg/m². Taxol is also used at high doses during bone marrow transplant, for example, at doses as high as 825 mg/m². In some embodiments, taxol treatment during bone marrow transplantation is in conjunction with one or more of the following agents: melphalan, cyclophosphamide, thiotepa and carboplatin. *See, e.g.,* Vahdat et al (2002) Bone Marrow Transplant 30(3):149-153.

The agonist anti-trkC antibody may be administered in conjunction with the taxol, *i.e.,* administered in combination with, in concert with, or sequentially with taxol. Such administration includes administering the antibody to the patient prior to administration of the neuropathy-inducing drug (such as taxol), administering the antibody to the patient during administration of the neuropathy-inducing drug, or administration of the antibody to the patient after the administration of the neuropathy-inducing drug. Administration in conjunction, as used herein, comprises simultaneous administration and/or administration at different times. Administration in conjunction also encompasses administration as a co-formulation (*i.e*., the agonist anti-trkC antibody and taxol are present (combined) in the same composition) and/or administration as separate compositions. As used herein, "administration in conjunction" is meant to encompass any circumstance wherein agonist anti-trkC antibody and taxol are administered in an effective amount to an individual. As further discussed herein, it is understood that the agonist anti-trkC antibody and taxol can be administered at different dosing frequencies and/or intervals. For example, an agonist anti-trkC antibody may be administered weekly, while taxol may be administered more frequently. It is understood that an agonist anti-trkC antibody and taxol can be administered using the same route of administration or different routes of administration, and that different dosing regimens may change over the course of administration(s). Administration may be before the onset of sensory neuropathy.

The treatment of cancer with taxol can also be enhanced as described herein, by administration of the taxol in conjunction with an agonist anti-trkC antibody. Administration of agonist anti-trkC antibody may permit increased taxol dosing, due to elimination of dose-limiting side effects of taxol treatment such as peripheral sensory neuropathy. The relative amounts and ratios of agonist anti-trkC antibody and taxol may vary. In some embodiments, enough agonist anti-trkC antibody will be administered so as to allow a reduction of undesired side-effects (such as sensory neuropathy) induced by or associated with taxol treatment.

### Methods of assessing efficacy of treatment with agonist anti-trkC antibodies

Assessment and diagnosis of taxol-induced sensory neuropathy is well known in the art. Assessment of treatment efficacy can be performed on several different levels. Assessment may be made by monitoring clinical signs, *e.g*., electrophysiological responses, or anatomical or molecular changes to affected neurons (including sensory neurons). *See, e.g.,* Quasthoff (2002) J. Neurology 249:9-17. In some embodiments, taxol-induced sensory neuropathy is characterized by any of the following symptoms: distal symmetrical paraesthesia, pall-hypaesthesia, loss of joint position sense, painful dysaesthesia, Lhermitte's sign, pain (including allodynia and/or hyperalgesia), progressive distal and/or proximal paresis, myalgia, paralytic ileus, orthostatic hypotension, and arrhythmia; and degeneration of peripheral sensory neurons (including large-fiber sensory neurons). As is known in the art, clinical assessment of taxol-induced sensory neuropathy includes, but is not limited to, any of a standard neurological examination, patient interview, or more specialized quantitative testing. These more specialized quantitative tests may include, but are not limited to, determination of conduction velocity of the affected neurons by, e.g. use of microneurography or other electrophysiological testing; quantitative and/or quantitative determination of ability to sense cutaneous stimulation, including, but not limited to, heat, light touch, vibration, or two point discrimination; tests of hearing; specialized tests of balance; specialized tests of proprioception, or kinesthetic sense; tests of autonomic function, including, but not limited to, test of blood pressure control; and tests of heart rate response to various physiological and pharmacological stimuli. These tests may also include tests of motor skill.

### Compositions for use in treatment of taxol-induced sensory neuropathy

The invention also provides compositions for use in any of the methods described herein. The compositions used in the methods of the invention comprise an effective amount of an agonist anti-trkC antibody. Examples of such compositions, as well as how to formulate, are also described in an earlier section and below. The invention also provides any of the compositions described for any use described herein whether in the context of use as medicament and/or use for manufacture of a medicament.

The composition used in the present invention can further comprise pharmaceutically acceptable carriers, excipients, or stabilizers (Remington: The Science and practice of Pharmacy 20th Ed. (2000) Lippincott Williams and Wilkins, Ed. K. E. Hoover.), in the form of lyophilized formulations or aqueous solutions. Acceptable carriers, excipients, or stabilizers are nontoxic to recipients at the dosages and concentrations, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (*e.g*. Zn-protein complexes); and/or nonionic surfactants such as TWEEN™, PLURONICS™ or polyethylene glycol (PEG). Pharmaceutically acceptable excipients are further described herein.

In one aspect, the invention provides compositions comprising an agonist anti-trkC antibody. In other embodiments, the agonist anti-trkC antibody recognizes human trkC. In still other embodiments, the agonist anti-trkC antibody is humanized (such as antibody A5 described herein). In other embodiments, the anti-trkC agonist antibody comprises one or more GDR(s) of antibody A5 (such as one, two three, four, five or, in some embodiments, all six CDRs from A5). In still other embodiments, the anti-trkC agonist antibody comprises the amino acid sequence of the heavy chain variable region shown in Table 1 (SEQ ID NO:1) and the amino acid sequence of the light chain variable region shown in Table 2 (SEQ ID NO:2). In still other embodiments, the agonist anti-trkC antibody is a human antibody.

It is understood that the compositions can comprise more than one agonist anti-trkC antibody (*e.g*., a mixture of agonist anti-trkC antibodies that recognize different epitopes of trkC). Other exemplary compositions comprise more than one agonist anti-trkC antibody that recognize the same epitope(s), or different species of agonist anti-trkC antibodies that bind to different epitopes of trkC.

The agonist anti-trkC antibody and compositions thereof can also be used in conjunction with other agents that serve to enhance and/or complement the effectiveness of the agonist anti-trkC antibody. For example, such additional compounds may include compounds known to be useful for the treatment of taxol-induced sensory neuropathy or side effects of taxol treatment, e.g. anemia or nausea, including but not limited to: erythropoietin (Epoiten, Darbopoiten) G-CSF, and GM-CSF, phenothiazines (Compazine), Zofran, and Anzemet. Such molecules are suitably present in combination in amounts that are effective for the purpose intended. The trkC agonist antibody and compositions thereof can also be used in conjunction with other agents that serve to enhance and/or complement the effectiveness of the antibodies, including erythropoietin (Epoiten, Darbopoiten) G-CSF, and GM-CSF, phenothiazines (Compazine), Zofran, and Anzemet.

### Kits

may be used in the instant methods. Kits include one or more containers comprising an anti-trkC agonist antibody and, in some embodiments, further comprise instructions for use in accordance with any of the methods of the invention described herein (such as methods for treating taxol-induced sensory neuropathy). These instructions may comprise a description of selecting an individual suitable for treatment based on identifying whether that individual has a taxol-induced sensory neuropathy and/or is at risk of developing taxol-induced sensory neuropathy, and may further describe administration of the trkC agonist antibody for treatment and/or prevention of the sensory neuropathy. Any of the kits described may be for any use described herein whether in the context of use as medicament and/or use for manufacture of a medicament.

Thus, kits may comprise an agonist anti-trkC antibody. kits for use with the methods described herein may comprise an agonist anti-trkC antibody. The instructions may comprise description of administering taxol in conjunction with an agonist anti-trkC antibody.

The kits are in suitable packaging. Suitable packaging include, but is not limited to, vials, bottles, jars, flexible packaging (*e.g*., sealed Mylar or plastic bags), and the like. The kit may comprise a container and a label or package insert(s) on or associated with the container. The label or package insert indicates that the composition is useful for treating, preventing or ameliorating taxol-induced neuropathy. Instructions may be provided for practicing any of the methods described herein. The container holds a composition which is effective for treating taxol-induced sensory neuropathy, and may have a sterile access port (for example the container may be an intravenous solution bag or a vial having a stopper pierceable by a hypodermic injection needle). At least one active agent in the composition is a trkC agonist antibody. The container may further comprise a second pharmaceutically active agent. Kits may optionally provide additional components such as buffers and interpretive information.

The following Examples are provided to illustrate but not limit the invention.

### EXAMPLES

### Examples 1. Effect of NT3 and agonist anti-trkC monoclonal antibodies on neuronal survival and axonal outgrowth

NT3 has been implicated in treatment of cis-platinum and pyridoxine-induced neuropathies. This example demonstrates that anti-trkC agonist antibodies did not mimic all of the biological activities of NT-3, the physiological ligand of the trkC receptor.
*Ability of NT-3 or agonist anti-trkC monoclonal antibody to promote neurite outgrowth of adult DRG neurons*

In order to test the ability of anti-trkC agonist antibodies to mimic the effects of NT-3 on adult sensory neurons, cultures of adult DRG neurons were grown in the presence or absence of various concentrations of NT-3 or the mouse agonist anti-trkC monoclonal antibody 2256. After culture for 48 hours, cultures were fixed and stained with RT97 antibodies, and neurite outgrowth was assessed in RT97+ neurons. As shown in Figure 1, treatment with NT-3 resulted in increased neurite outgrowth, while treatment with an agonist anti-trkC antibody did not result in significant outgrowth.

Dorsal root ganglia were dissected from adult (6 months old) Sprague-Dawley rats, and dissociated and cultured by standard techniques (Lindsay, 1988, J Neurosci. Jul; 8(7):2394-405). Briefly, ganglia were stripped of their sheaths, and incubated in collagenase for two 90 minute periods at 37 degrees. They were then washed extensively and incubated in trypsin for 30 minutes. After a further washing, the ganglia were dissociated by gentle trituration through a flame polished Pasteur pipet. Neurons were then enriched in this mixture by sedimentation through a 30% Percoll (Pharmacia) gradient as described (Horie, 1994, NeuroReport 6, 37-40). Enriched neurons were plated at a density of 6.25/mm2 on polyornithine and laminin coated 96-well plates. Medium consisted of F-14 with additives 2mM glutamine, 0.35% Albumax II (Gibco-BRL), 60 ng/ml progesterone, 16ug/ml putrescine, 400 ng/ml L-thyroxine, 38 ng/ml sodium selenite, 340 ng/ml tri-iodo-thyronine, 60 ug/ml penicillin and 100 ug/ml streptomycin and the appropriate test factors added at the indicated concentrations. After forty-eight hours of culture, the cells were fixed with 4% formaldehyde in PBS with 5% sucrose for 16-24 hours at 4 degrees centigrade.

Staining with antibody RT-97 was used to indicate the subclass of DRG neurons with large cell bodies and which, *in vivo,* would have a large diameter myelinated axon. This class preferentially expressed trkC. Cultures were rinsed 3 times with PBS and 3 times with 0.1M Tris solution containing 0.1% gelatin, 0.9% NaCl, and 0.3% triton (TGST). Cultures were then incubated overnight in RT-97 antibody at a concentration of 1/100 in TGST containing 5% sucrose and 4% normal donkey serum. This incubation was terminated with extensive washing of the cultures in TGST and a Cy-3 conjugated goat antimouse IgG secondary antibody was added to the cells at a dilution of 1:400 for four hours in TGST containing 5% sucrose and 4% normal donkey serum. After a further extensive washing in TGST, the cells were examined and images digitally captured with fluorescent optics with a 4x objective. These images were analyzed to selectively indicate the processes of neurons by using thresholding, binarizing, skeletonization and measurement of the resulting pixel area of neuronal processes. The results, shown in Figure 1, are presented as the mean pixel area of neuronal processes (+/- the standard error of the mean) (n=4). Treatment with NT-3 resulted in increased neurite outgrowth, while treatment with an agonist anti trkC antibody did not result in significant outgrowth.

### Ability of NT-3 and agonist anti-trkC monoclonal antibody to promote survival of embryonic rat trigeminal neurons.

The trigeminal ganglion (TG) is comprised of cutaneous sensory neurons innervating the facial region. An appropriate age to assay an anti-trkC agonist antibody for survival promoting activity on mouse trigeminal neurons is embryonic day 11 (E11), as at this age the neurons express TrkC, respond to NT3 in culture and cannot survive in the absence of neurotrophic support. An equivalent age in the rat is E12.

Dissociated cultures of TG neurons were established from E11 Swiss-Webster mice or E12 Sprague Dawley rats. Dissected ganglia were trypsinized and dissociated by trituration (Davies et al., 1993, Neuron 11, 565-574). The neurons were plated at low density in 35mm diameter tissue culture Petri dishes in a defined, serum-free medium on a polyornithine/laminin substratum. BDNF (2ng/ml) was added as a positive control and some neurons were grown in the absence of factors as a negative control. NT3 (2,0.4 and 0.08ng/ml) and the mouse anti-trkC agonist antibody, 2256 (*see* PCT Publication No. WO 01/98361) (2, 0.4 and 0.08 µg/ml) was added at the time of plating, at varying concentrations, in duplicate. To quantify the number of neurons surviving under different experimental conditions the total number of neurons was counted 4-6 hours after plating and again after 24 and 48 hours. The number of neurons at 24 and 48 hours was expressed as a percentage of the 6 hour count.

The results of this experiment are shown in Figure 2. Treatment with NT-3, as well as agonist anti-trkC antibody, resulted in survival of trigeminal neurons. BDNF promoted the survival of the majority of E11 mouse and E12 rat trigeminal ("TG") neurons, which otherwise died in the absence of factors. After 24 hours in culture, concentrations of NT3 as low as 0.08 ng/ml promoted the survival of all the neurons suggesting that this was a trkC-mediated event. Only a subset of mouse TG neurons were rescued by NT3 after 48 hours in culture whereas in cultures of rat TG neurons the majority of the neurons survived in the presence of NT3 after 48 hours. This may have partly reflect the overall better health of the rat TG neurons as evidenced by the greater survival of rat control cultures compared to mouse. The anti-trkC agonist antibodies were able to promote the survival of both E11 mouse and E12 rat TG neurons.

### Example 2: Development and dose dependence of taxol-induced sensory neuropathy in experimental animal model

Development and dose dependency of taxol-induced sensory neuropathy was examined in adult rats. Taxol was prepared as a solution in Cremophor/ethanol (50:50), which was diluted with saline (1 part Cremophor/ethanol to four parts saline) immediately before dosing. Adult rats were treated by slow IV infusion on day 1 and day 4 of the study. One group received 12 mg/kg of taxol, one group received 15 mg/kg of taxol, one group received 18 mg/kg of taxol, and one group received vehicle alone. Large fiber sensory neuron function was tested at day 0, day 14, and day 28 using electrophysiological methods essentially as described in Cliffer et al., Ann. Neurol (1998) 43:46-55. Specifically, H-wave (reflex sensory) response, sensory nerve conduction velocity, and the ratio of H wave compound action potential and M wave compound action potential were measured by recording in foot muscle following stimulation of the sciatic nerve at the thigh and the calf.

### Sciatic recording

Sciatic nerve recording was conducted as follows: stainless steel needles were used as stimulating (26-gauge), recording (28-gauge) and grounding electrodes. For sciatic stimulation, the anode was inserted at the sciatic notch and the cathode 1 cm distal to the anode. For tibial stimulation, the cathode was inserted at the ankle (Achilles tendon) and the anode 1 cm proximal to the cathode. Recording electrodes were placed on the foot, the active electrode into the abductor hallucis muscles and a reference electrode on the base of the 5^{th} phalanx. A ground electrode was placed at the base of the tail. Biphasic square-wave stimuli of constant current and 0.2 ms of duration were delivery at a frequency of 0.2-0.5 Hz by means of an isolated pulse stimulator (A-M Systems, Model 2100). Recorded outputs were differentially amplified (Brownlee Precision, Model 210A), and digitally acquired at 20,000- 40,000 samples/sec (AD Instruments, PowerLab/4SP) and stored in a computer for later analysis.

Stimulating electrode positions were adjusted to achieve threshold for the M or H waves and stimulation intensity was varied to maximize the H-wave amplitude (Hmax) and the M-wave amplitude (Mmax). The distance between the sciatic and the tibial cathodes was divided by the difference in M and H-wave latencies from the two locations (to the first major negative peaks) to calculate motor and sensory conduction velocities. Peak-to-peak amplitudes were measured for the M and the H waves, and the Hmax/Mmax ratio was calculated to provide a measurement of the H-wave amplitude normalized to the M-wave amplitude.

### Caudal nerve recording

Caudal nerve recording and stimulation were performed using bare 26-gauge stainless steel electrodes inserted subcutaneously on the side of the tail. A ground electrode was always placed at equal distance of the stimulation and the recording electrodes. For stimulation, monophasic square pulses of 0.2 ms duration and 0.8-1 mA amplitude at 0.5 Hz were used. The distance between the recording electrodes was constant (10 mm). Two configurations were used (A and B) and at each of them stimulation were done at two distances from recording electrodes:

A) Stimulation electrodes proximal, recording electrodes distal: This configuration tested motor function in the tail. For the first measurement, the cathode was placed at 30 mm from the tail base and the anode 1 cm proximal. For the second measurement, the stimulation electrodes were moved 30-35 mm distal. In both cases the recording electrodes were placed 110-130 mm from the base of the tail.

B) Stimulation electrodes distal, recording electrodes proximal: This configuration tested sensory function in the tail. The recording electrodes were placed at 30 mm from the tail base. For the first measurement (S1), the stimulation electrodes were placed at approximately 110-130 mm from the base of the tail. For the second measurement (S2), the stimulation electrodes were placed approximately 30-35 mm more proximal. Nerve conduction velocity was obtained by measuring the distance between two stimulation points and dividing the results by the difference in latency between the proximal and distal stimulation points.

### Results

Treatment with 12 mg/kg and 18 mg/kg of taxol resulted in reduced sensory function. Specifically, electrophysiological testing revealed reduced tibial and sciatic H wave amplitude (Figs. 3A and 3B, respectively), reduced sciatic nerve conduction velocity (Fig. 3C), reduced caudal nerve H wave amplitude (Figs. 4A and 4B, respectively), but not reduced caudal sensory nerve conduction velocity (Fig. 4C). Treatment with 15 mg/ml taxol also resulted in reduced sensory function (data not shown). The severity of the neuropathy was also dose-dependent *(see* Figs. 3A-3C and 4A-4C). Taxol dosing of 12 mg/kg and 15 mg/kg were selected for further experimentation, based on the rapidity and depth of neuropathy obtained at these doses.

### Example 3. Effect of agonist anti-trkC monoclonal antibodies on taxol-induced sensory neuropathy in a rat animal model.

We tested the ability of an agonist anti-trkC monoclonal antibody to protect from the sensory neuropathy induced by taxol treatment. Adult rats were treated intravenously with 5 mg/kg of the mouse agonist anti-trkC antibody 2256 (*see* PCT Publication No. WO 01/98361) on days 0 and day 7 of the study. 15 mg/kg of taxol was administered by slow IV infusion on days 1 and 4. The function of the caudal nerve was tested electrophysiologically at day 14 according to the method described above. Briefly, the M-wave (direct motor) and H-wave (reflex sensory) response was recorded after stimulation of the caudal nerve as described herein. The results were shown as the ratio of the sensory compound action potential amplitude to the motor compound action potential amplitude. As shown in Figure 5, treatment with anti-trkC agonist antibody prevented the taxol-induced loss of compound action potential amplitude. This indicates that treatment with an anti-trkC agonist antibody ameliorated sensory neuropathy induced by taxol treatment in a rat model. Secondary rat antibodies to the mouse agonist anti-trkC antibody were observed after about ten days after initiation of antibody treatment.

### Example 4: Development of taxol-induced sensory neuropathy in a mouse model, and effect of agonist anti-trkC monoclonal antibodies on taxol-induced sensory neuropathy.

This example describes a mouse model for taxol-induced sensory neuropathy and demonstrates that treatment with an agonist anti-trkC antibody ameliorated sensory neuropathy induced by taxol treatment in this model. The mouse model provided an advantage in that the mouse anti-trkC agonist antibodies are species compatible and thus did not provoke an undesired immune response when administered.

Female Swiss-Webster mice (8 weeks old) were acclimated to the facility for one week before dosing began. Animals were treated with vehicle alone (control), taxol alone, or agonist anti-trkC antibody and taxol. Taxol was obtained from Calbiochem and prepared as a 20 mg/kg solution in Cremophor/ethanol (50:50 w/w), which was diluted with saline (1 part Cremophor/ethanol to four parts saline vol:vol), filtered through a 0.1 micron filter and administered within 20 minutes of preparation. All treatment animals received one round of three taxol or control (vehicle) treatments every other day, e.g. Monday, Wednesday and Friday. The total taxol dose administered to each animal was 300 mg/m², split evenly into the three doses described above. Taxol dose was determined on a mg/m² basis (using the formula that body surface area (in square cm) equals 10.5 times the weight in grams to the two-thirds power). Mouse anti-trkC agonist antibody 2256 was administered at weekly intervals, starting with the day of the first taxol dose. Taxol was administered IP and anti-trkC antibody was administered subcutaneously in the scruff. All dosing was under light isoflurane anaesthesia. For the week following the week of taxol treatment, prophylactic antibiotics were provided in the food to prevent potential opportunistic infections associated with taxol-induced transient immune depression as follows: sulfamethoxazole (60 mg) and trimethoprim (10 mg) per five gram food tablet, per day, (Bio-Serv product S0443-J).

Large fiber sensory neuron function was tested at day 14 using electrophysiological methods as described below. Specifically, the ratio of H wave CAP and M wave CAP were determined in the tail after stimulation of the caudal nerve as described below.

### Caudal nerve recording

All recordings and analysis were performed by experimenters blinded to the experimental treatment of the animals. Animals were anaesthetized with ketamine/Xylazine 60 mg/kg ketamine IP plus 5 mg/kg xylazine IP, and a rectal temperature of 36-37 degrees was maintained throughout the session by keeping the animal on a warm pad (deltaphase). Recording and stimulation were done through bare 26-gauge stainless steel electrodes inserted subcutaneously on the side of the tail. A ground electrode was always placed at equal distance from the stimulation and the recording electrodes. Electrodes were disinfected with RX-444 (or other suitable disinfectant) and were rinsed with water between animals. For stimulation, monophasic square pulses of 0.2 ms duration and 0.8-1 mA amplitude at 0.5 Hz were used. The distance within the recording electrodes was constant (10 mm).

To study the motor and the sensory components of the nerve, two configuration are used (A and B) and at each of them stimulation was done at two distances from recording electrodes.

(A) Stimulation electrodes proximal, recording electrodes distal. This configuration tested motor function in the tail. For the first measurement (S1) the stimulating cathode was placed at 15 mm from the tail base and the anode 1 cm proximal to the cathode. For the second measurement (S2), the stimulation electrodes were moved 10-15 mm distal. For both measurements, the recording electrodes were placed 35 mm from the base of the tail.

(B) Stimulation electrodes distal, recording electrode proximal. This configuration tested sensory function in the tail. The recordings electrodes were placed 5 mm from the tail base and the stimulation electrodes were placed at first at 35 mm from the base of the tail, and second at a position that was 10-15 mm more proximal.

### Results

As shown in Figure 6, there was a significant reduction in action potential size in animals treated with taxol and this was prevented by treatment with 2 mg/kg of trkC agonist antibody. Thus, treatment with an agonist anti-trkC antibody ameliorated sensory neuropathy induced by taxol treatment. Group size; n=10 for vehicle, n=5 for taxol alone, and n=8 for taxol plus antibody.

### Example 5: Effect of mouse trkC agonist antibody 2256 on taxol-induced allodynia

This experiment tested the effectiveness of agonist anti-trkC antibody in relieving the symptoms of neuropathic pain produced by taxol treatment, namely mechanical allodynia. Rats treated with taxol develop allodynia. *See, e.g.,* Poloman et al., Pain (2001) 94(3):293-304; Dina et al (2001) Neuroscience 108(3):507-15. When rats were treated with taxol, they developed a long lasting mechanical allodynia (*i.e*., increased noxious sensation) to a normally non-noxious stimulus). Treatment with anti-trkC agonist antibody 2256 ameliorated taxol-induced allodynia. Further, animals treated with trkC agonist antibody and taxol showed faster recovery from taxol-induced allodynia, than animals treated with taxol alone.

Experiments were performed on male Sprague-Dawley rats (Harlan, Oregon WI). 50 rats were acclimated to the facility for seven days, and acclimated to the von Frey testing chambers (plastic cages with a wire mesh bottom which allowed full access to the paws), for a further two days before testing.

Allodynia to mechanical stimulus (touch) was tested using a standard series of von Frey hairs (nylon monofilaments) to assess mechanical withdrawal threshold, using the up and down method described by Chaplan et al., J. Neurosci. Methods, 53(1):55-63 (1994). After two baseline testing sessions, thirty-eight rats were selected for the study, based on consistent results between the two baseline testing sessions and consistent results between right and left paws. These rats were split into two groups of thirteen animals each (for treatment with taxol or taxol plus mAb 2256), and one group of twelve (for treatment with vehicle (control)) animals. The groups were balanced for their baseline response thresholds and body weight.

At day minus two ("-2"), one group of thirteen animals received 2mg/kg of mouse anti-trkC agonist antibody mAb 2256 injected intraperitoneally. These antibody injections were repeated weekly throughout the duration of the study.

On day zero, animals began their treatment with taxol or vehicle, essentially as described by Palomino, *supra.* Clinical grade taxol (obtained as a 6mg/ml stock in a vehicle comprising absolute ethanol and Cremophore EL^{®} (50:50, v:v); Mead Johnson Oncology Products, a division of Bristol Myers Squibb). Immediately before use, the taxol stock solution was diluted with dextrose saline to Img/ml and sterile filtered through a 0.2 micron filter. Similarly diluted and filtered Cremophor EL^{®}/ethanol solution was used as a control in the vehicle group.

Taxol and control solution were administered essentially as described in Polomano et al., Pain 94(3):293-304 (2001) by an investigator blinded to the antibody treatment status of the animals. Specifically, in days 0, 2, 4, and 6, both groups of thirteen animals (with and without mAb 2256 treatment) were injected with 1.0 mg/kg taxol intraperitoneally. The group of twelve animals (the vehicle group) was injected with a volume-matched control solution based on the same dosing schedule.

At intervals after taxol treatment (every seven to eleven days), measurements of mechanical threshold (*i.e*., mechanical allodynia) were carried out by an investigator blind to the treatment of each animal and any group assignments. Measurements of withdrawal threshold of the right and left paws were averaged to obtain a measure of the overall withdraw threshold. This was expressed as the percent of the average threshold obtained for each animal before taxol treatment *i.e*., the baseline, as presented in Figure 7. Two way analysis of variance (ANOVA) was used for statistic analysis of the data.

As shown in Figure 7, rats treated with taxol alone developed a significant and long-lasting decrease in the response threshold of animals, indicating allodynia (p < 0.0001 using 2-way ANOVA analysis). Cotreatment of animals with taxol and agonist anti-trkC antibody decreased the depth of allodynia observed with taxol treated animals, and dramatically increased the speed of recovery from the allodynia (p< 0.05, using 2-way ANOVA analysis).

Despite effort to standardize the rodent models of taxol-induced neuropathy, these models displayed variability. Specifically, in the mouse model, of three attempts to generate an electrophysiologically detectable sensory neuropathy by giving taxol as described in this application, only one gave a statistically significant neuropathy. The experiment that did generate a neuropathy is shown here as an example, and the animals treated with taxol plus agonist anti-trkC antibody showed a significant protection from the taxol effect. The lack of neuropathy in the next two attempts may have been due to changing the source of the animals for the two experiments where there was not a significant neuropathy generated (*i.e*., the animals in the second and third experiments were from a different source than the animals from the first experiment) due to an outbreak of pathogen at the supplier of mice for the first experiment. In the rat model using electrophysiology as an endpoint, there was a variable effect of treating rats with taxol. In additional to the experiments described here, there were two experiments where no significant neuropathy was detected. In addition, there were two experiments in which a significant neuropathy was generated, and a trend towards amelioration of the neuropathy was observed in antibody-treated animals, but the effect of the trkC agonist antibody treatment did not reach the p<0.05 standard for statistical significance. In the rat model using allodynia as an endpoint, out of a total of six experiments attempting to generate a state of allodynia due to taxol treatment, two successfully generated rats with allodynia. One of these is the experiment shown here as an example (where trkC agonist antibody treatment ameliorated allodynia) and the other was the first experiment to test the model and determine if allodynia would be detectable. In this experiment, there was no group of rats treated with antibody, so no conclusion could be drawn from it about the potential efficacy of trkC agonist treatment.

## Claims

1. An anti-trkC agonist antibody for treating taxane-induced sensory neuropathy in an individual.

2. The antibody of claim 1, wherein taxane-induced neuropathic pain is treated.

3. The antibody of claim 2, wherein the neuropathic pain comprises allodynia.

4. The antibody of claim 1, wherein the anti-trkC agonist antibody binds human trkC and rodent trkC.

5. The antibody of any one of the preceding claims, wherein the anti-trkC agonist antibody binds a continuous or discontinuous epitope in domain 4 and/or domain 5 of trkC.

6. The antibody of any one of the preceding claims, wherein the anti-trkC agonist antibody is a human antibody.

7. The antibody of any one of the preceding claims, wherein the anti-trkC agonist antibody is a humanized antibody.

8. The antibody of any one of the preceding claims, wherein the antibody is a monoclonal antibody.

9. A pharmaceutical composition for treating taxane-induced sensory neuropathy comprising an effective amount of an anti-trkC agonist antibody, and a pharmaceutically acceptable carrier.

## Patentansprüche

1. Anti-trkC-Agonist-Antikörper zur Behandlung von Taxan-induzierter sensorischer Neuropathie in einem Individuum.

2. Antikörper gemäss Anspruch 1, worin Taxan-induzierter neuropathischer Schmerz behandelt wird.

3. Antikörper gemäss Anspruch 2, worin der neuropathische Schmerz Allodynie umfasst.

4. Antikörper gemäss Anspruch 1, worin der Anti-trkC-Agonist-Antikörper humanes trkC und Nagetier-trkC bindet.

5. Antikörper gemäss irgendeinem der vorangehenden Ansprüche, worin der Anti-trkC-Agonist-Antikörper ein zusammenhängendes oder unterbrochenes Epitop in Domäne 4 und/oder Domäne 5 von trkC bindet.

6. Antikörper gemäss irgendeinem der vorangehenden Ansprüche, worin der Anti-trkC-Agonist-Antikörper ein humaner Antikörper ist.

7. Antikörper gemäss irgendeinem der vorangehenden Ansprüche, worin der Anti-trkC-Agonist-Antikörper ein humanisierter Antikörper ist.

8. Antikörper gemäss irgendeinem der vorangehenden Ansprüche, worin der Antikörper ein monoklonaler Antikörper ist.

9. Pharmazeutische Zusammensetzung zur Behandlung von Taxan-induzierter sensorischer Neuropathie, die eine wirksame Menge eines Anti-trkC-Agonist-Antikörpers und einen pharmazeutisch annehmbaren Träger umfasst.

## Revendications

1. Anticorps agoniste anti-trkC pour le traitement d'une neuropathie sensorielle induite par le taxane chez un individu.

2. Anticorps selon la revendication 1, dans lequel la douleur neuropathique induite par le taxane est traitée.

3. Anticorps selon la revendication 2, dans lequel la douleur neuropathique comprend l'allodynie.

4. Anticorps selon la revendication 1, dans lequel l'anticorps agoniste anti-trkC se lie au trkC humain et au trkC du rongeur.

5. Anticorps selon l'une quelconque des revendications précédentes, dans lequel l'anticorps agoniste anti-trkC se lie à un épitope continu ou discontinu dans le domaine 4 et/ou le domaine 5 du trkC.

6. Anticorps selon l'une quelconque des revendications précédentes, dans lequel l'anticorps agoniste anti-trkC est un anticorps humain.

7. Anticorps selon l'une quelconque des revendications précédentes, dans lequel l'anticorps agoniste anti-trkC est un anticorps humanisé.

8. Anticorps selon l'une quelconque des revendications précédentes, dans lequel l'anticorps est un anticorps monoclonal.

9. Composition pharmaceutique pour le traitement de la neuropathie sensorielle induite par le taxane comprenant une quantité efficace d'un anticorps agoniste anti-trkC et un véhicule pharmaceutiquement acceptable.


### SEQUENCE LISTING

<110> RINAT NEUROSCIENCE CORP.
<120> METHODS FOR TREATING TAXOL-INDUCED SENSORY NEUROPATHY
<130> N.95095 SER
<140> EP 03814397.0
   <141> 2003-12-23
<150> PCT/US03/041367
   <151> 2003-12-23
<150> US 60/436,147
   <151> 2002-12-23
<160> 2
<170> FastSEQ for Windows Version 4.0
<210> 1
   <211> 123
   <212> PRT
   <213> Artificial Sequence
<220>
   <223> Synthetic Construct
<400> 1
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Ser Tyr
               20                  25                  30
   Arg Ile His Trp Val Arg Gln Ala Pro Gly Gln Gly Leu Glu Trp Met
           35                  40                  45
   Gly Glu Ile Tyr Pro Ser Asn Ala Arg Thr Asn Tyr Asn Glu Lys Phe
       50                  55                  60
   Lys Ser Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Lys Tyr Tyr Tyr Gly Asn Thr Arg Arg Ser Trp Tyr Phe Asp
               100                 105                 110
   Val Trp Gly Gln Gly Thr Thr Val Thr Val Ser
           115                 120
<210> 2
   <211> 113
   <212> PRT
   <213> Artificial Sequence
<220>
   <223> Synthetic Construct
<400> 2
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Glu Ser Ile Asp Asn Tyr
               20                  25                  30
   Gly Ile Ser Phe Leu Ala Trp Tyr Gln Gln Lys Pro Gly Lys Ala Pro
           35                  40                  45
   Lys Leu Leu Ile Tyr Ala Ala Ser Asn Arg Gly Ser Gly Val Pro Ser
       50                  55                  60
   Arg Phe Ser Gly Ser Gly Ser Gly Thr Asp Phe Thr Phe Thr Ile Ser
   65                  70                  75                  80
   Ser Leu Gln Pro Glu Asp Ile Ala Thr Tyr Tyr Cys Gln Gln Ser Lys
                   85                  90                  95
   Thr Val Pro Arg Thr Phe Gly Gln Gly Thr Lys Leu Glu Ile Lys Arg
               100                 105                 110
   Thr